(19) Europäisches Patentamt
European Patent Office
Office européen des brevets

(11) EP 3 087 395 B1

# (12) EUROPEAN PATENT SPECIFICATION

(45) Date of publication and mention of the grant of the patent:
**06.12.2017 Bulletin 2017/49**

(21) Application number: **13815518.9**

(22) Date of filing: **23.12.2013**

(51) Int Cl.:
***G01N 33/68*** *(2006.01)*

(86) International application number:
**PCT/EP2013/077955**

(87) International publication number:
**WO 2015/096858 (02.07.2015 Gazette 2015/26)**

## (54) METHOD FOR DETECTING A SYSTEMIC INFLAMMATION AND TEST SYSTEM

VERFAHREN UND VORRICHTUNG ZUM NACHWEIS EINER SYSTEMISCHEN ENTZÜNDUNG

PROCÉDÉ POUR DÉTECTER UNE INFLAMMATION SYSTÉMIQUE, ET SYSTÈME D'ANALYSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**02.11.2016 Bulletin 2016/44**

(73) Proprietor: **Mediagnost Gesellschaft für Forschung und Herstellung von Diagnostika GmbH**
**72127 Kusterdingen (DE)**

(72) Inventor: **KAUFMANN, Ines**
**85653 Aying (DE)**

(74) Representative: **Louis Pöhlau Lohrentz**
**Patentanwälte**
**Postfach 30 55**
**90014 Nürnberg (DE)**

(56) References cited:
**US-A1- 2004 097 460**

- **VERCELLINO M ET AL: "Progranulin expression in brain tissue and cerebrospinal fluid levels in multiple sclerosis", MULTIPLE SCLEROSIS, SAGE PUBLICATIONS, BASINGSTOKE, GB, vol. 17, no. 10, 1 November 2011 (2011-11-01), pages 1194-1201, XP009179969, ISSN: 1352-4585, DOI: 10.1177/1352458511406164 [retrieved on 2011-05-25]**
- **HOSSEIN-NEZHAD A ET AL: "Obesity, inflammation and resting energy expenditure: Possible mechanism of progranulin in this pathway", MINERVA ENDOCRINOLOGICA, MINERVA MEDICA, TORINO, IT, vol. 37, no. 3, 1 September 2012 (2012-09-01), pages 255-266, XP009179976, ISSN: 0391-1977**
- **J. JIAN ET AL: "Insights into the role of progranulin in immunity, infection, and inflammation", JOURNAL OF LEUKOCYTE BIOLOGY, vol. 93, no. 2, 22 October 2012 (2012-10-22), pages 199-208, XP55138274, US ISSN: 0741-5400, DOI: 10.1189/jlb.0812429**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention is directed to a method for detecting a systemic inflammation and a use of a test system for detecting a systemic inflammation in an isolated sample of an individual, as well as a use of an array comprising detection molecules for detecting a systemic inflammation in an isolated sample of an individual. The present invention is also directed to a method for determining whether a compound is effective in the treatment of a systemic inflammation. The present invention is further directed to progranulin for use as a biomarker for a systemic inflammation.

A systemic inflammation is an inflammation affecting the whole body.

A systemic inflammation may be caused by the immune system's response to a serious infection, most commonly bacteria, but also fungi, viruses, and parasites in the blood, urinary tract, lungs, skin, or other tissues of an individual.

A systemic inflammation can continue even after the infection that caused the inflammation is overcome.

Common symptoms of a systemic inflammation include those related to a specific infection, but usually accompanied by high fevers, hot, flushed skin, elevated heart rate, hyperventilation, altered mental status, swelling, and low blood pressure. In the very young and elderly people, or in people with weakened immune systems, the pattern of symptoms may be atypical, with hypothermia and without an easily localizable infection.

A systemic inflammation may be treated by intravenous administration of fluids and antibiotics. If fluid replacement isn't sufficient to maintain blood pressure, vasopressors can be used. Mechanical ventilation and dialysis may be needed to support the function of the lungs and kidneys, respectively.

Systemic inflammations include systemic inflammatory response syndrome (SIRS), sepsis, severe sepsis or septic shock.

Criteria for diagnosing a systemic inflammatory response syndrome (SIRS) were established in 1992 as part of the American College of Chest Physicians/Society of Critical Care Medicine Consensus Conference (American College of Chest Physicians/Society of Critical Care Medicine Consensus Conference: Crit Care Med. 1992, pages 864-874; Bone RC et al.: Crit Care Med. 1992, pages 724-726).

The conference concluded that the manifestations of SIRS include, but are not limited to:

- Body temperature less than 36°C(96.8°F) or greater than 38°C(100.4°F)
- Heart rate greater than 90 beats per minute
- Tachypnea (high respiratory rate), with greater than 20 breaths per minute; or, an arterial partial pressure of carbon dioxide less than 4.3 kPa (32 mmHg)
- leukocytes less than 4000 cells/$mm^3$ (4 x $10^9$ cells/L) or greater than 12,000 cells/$mm^3$ (12 x $10^9$ cells/L); or the presence of greater than 10% immature neutrophils (band forms).

SIRS can be diagnosed when two or more of these criteria are present.

Fever and leukocytosis are features of the acute-phase reaction. Tachypnea may be related to the increased metabolic stress due to infection and inflammation, but may also be an ominous sign of inadequate perfusion resulting in the onset of anaerobic cellular metabolism.

As an alternative, when two or more of the systemic inflammatory response syndrome criteria are met without evidence of infection, patients may be diagnosed simply with "SIRS." Patients with SIRS and acute organ dysfunction may be termed "severe SIRS."

Sepsis is a condition in which individuals both meet criteria for SIRS and have a known or highly suspected infection.

Severe sepsis is a sepsis complicated by organ dysfunction. Septic shock is a sepsis complicated by a high lactate level or by shock that does not improve after fluid resuscitation.

Systemic inflammations such as systemic inflammatory response syndrome (SIRS), sepsis, severe sepsis or septic shock have a worldwide incidence of more than 20 million cases a year, with mortality due to septic shock reaching up to 50 percent even in industrialized countries.

The mortality rate from sepsis, for example, is approximately 40% in adults, and 25% in children, and is significantly greater when left untreated for more than 7 days.

It is therefore an object of the present invention to provide means allowing an early detection of a systemic inflammation, especially a systemic inflammatory response syndrome (SIRS), a sepsis, a severe sepsis or a septic shock.

The object underlying the present invention is solved by providing a method for detecting a systemic inflammation comprising:

a) providing an isolated sample which has been taken from an individual,
b) determining a pathological level of progranulin in said isolated sample.

In a preferred embodiment of the method for detecting a systemic inflammation said systemic inflammation is a systemic inflammatory response syndrome (SIRS), a sepsis, a severe sepsis or a septic shock.

The level of progranulin in said isolated sample is preferably determined by immunological methods, proteomic techniques, gene expression analysis, and/or mass spectroscopy, preferably immunological methods, proteomic techniques, and/or mass spectroscopy.

The object of the present invention is further solved by a method of monitoring the development and/or the course and/or the treatment of a systemic inflammation comprising:

a) providing a first sample isolated from an individual at a first time,
b) determining a level of progranulin in said first sample,
c) providing at least a second sample isolated from said individual at a second time, wherein said second time is later than said first time,
d) determining the level of progranulin in said isolated sample,
e) comparing the determined level of progranulin in said second sample to the level of progranulin in said first sample,

wherein an increase of the level of progranulin in said second sample compared to the level of progranulin in said first sample is indicative for the progression of a systemic inflammation, and/or the course worsening and/or the treatment being ineffective, and
wherein a decrease of the level of progranulin in said second sample compared to the level of progranulin in said first sample is indicative for slowing down of the progression of a systemic inflammation, and/or the course improving and/or the treatment being effective.

In a preferred embodiment of the method of monitoring the development and/or the course and/or the treatment of a systemic inflammation said systemic inflammation is a systemic inflammatory response syndrome (SIRS), a sepsis, a severe sepsis or a septic shock.

In a preferred embodiment of the method of monitoring the development and/or the course and/or the treatment of a systemic inflammation said level of progranulin in said first sample and/or said second sample is determined by immunological methods, proteomics techniques, gene expression analysis, and/or mass spectroscopy, preferably immunological methods, proteomic techniques, and/or mass spectroscopy.

In a preferred embodiment of the method of monitoring the development and/or the course and/or the treatment of a systemic inflammation said second time is between 15 minutes to 48 hours, preferably between 30 minutes to 36 hours, preferably between 45 minutes to 18 hours, preferably between 1 hour to 24 hours, later than said first time.

In a further preferred embodiment of the method of monitoring the development and/or the course and/or the treatment of a systemic inflammation said second time is between 6 hours to 48 hours, preferably between 12 to 36 hours, preferably between 18 hours to 24 hours, later than said first time.

The object of the present invention is solved as well by a use of a test system for detecting a systemic inflammation in an isolated sample of an individual comprising:

a) at least one detection molecule, which specifically recognizes progranulin,
b) a reagent for detecting the binding of progranulin to said at least one detection molecule, and
c)optionally, a solid support which supports said at least one detection molecule.

In a further preferred embodiment of the use of a test system for detecting a systemic inflammation said systemic inflammation is a systemic inflammatory response syndrome (SIRS), a sepsis, a severe sepsis or a septic shock.

In a further preferred embodiment of the use of a test system for detecting a systemic inflammation said detection molecule is selected from the group consisting of an antibody against progranulin, an antibody fragment, which specifically binds to an epitope or a suitable structural element of progranulin, a receptor, which specifically binds to an epitope or a suitable structural element of progranulin, and mixtures thereof.

In a further preferred embodiment of the use of a test system for detecting a systemic inflammation said test system is in the form of an array, preferably a biochip, a 96 well plate or the like.

The object is solved as well by providing a method for determining whether a compound is effective in the treatment of a systemic inflammation comprising:

a) providing an isolated sample from an individual diseased of systemic inflammation and treated with a compound,
b) determining the level of progranulin in said isolated sample of said individual, and
c) comparing the determined level of progranulin with one or more reference values,

wherein an increase of the level of progranulin in said second sample compared to the level of progranulin in said first sample is indicative for the compound being ineffective for the treatment of a systemic inflammation, and wherein a decrease of the level of progranulin in said second sample compared to the level of progranulin in said first sample is indicative
for the compound being effective for the treatment of a systemic inflammation, and.

In a preferred embodiment of the method for determining whether a compound is effective in the treatment of a systemic inflammation an individual diseased of systemic inflammation is treated with a compound to be tested.

In a further preferred embodiment of the method for determining whether a compound is effective in the treatment of a systemic inflammation said systemic inflammation is a systemic inflammatory response syndrome (SIRS), a sepsis, a severe sepsis or a septic shock.

The object of the present invention is furthermore solved by a use of progranulin as biomarker for a systemic inflammation.

In a further preferred embodiment of the use of progranulin as biomarker for a systemic inflammation said systemic inflammation is a systemic inflammatory response syndrome (SIRS), a sepsis, a severe sepsis or a septic shock.

Further preferred embodiments are specified in the dependent claims.

Progranulin is an 68.5 kDa protein calculated on the basis of the amino acid sequence of progranulin, which consists of 593 amino acids including a N-terminal signal peptide of 17 amino acids.

The amino acid sequence of human progranulin is accessible via: UniProtKB/Swiss-Prot accession number: P28799 or
NCBI accession number: NP_002078.

The term "fragment of progranulin" means a fragment of the full length progranulin, i.e. including signal peptide, or of the mature progranulin, i.e. without signal peptide.

The term "fragment of progranulin" preferably means a fragment of the full length progranulin comprising a stretch of at least 8 amino acids, preferably at least 18 amino acids, preferably at least 29 amino acids, preferably at least 54 amino acids, preferably at least 68 amino acids, preferably at least 75 amino acids, preferably at least 89 amino acids, preferably at least 105 amino acids, more preferably at least 175 amino acids, of SEQ ID No. 1 or a stretch of at least 8 amino acids, preferably at least 18 amino acids, preferably at least 29 amino acids, preferably at least 54 amino acids, at least 68 amino acids, preferably at least 75 amino acids, preferably at least 89 amino acids, preferably at least 105 amino acids, more preferably at least 175 amino acids, of SEQ ID No. 1 having at least 90%, preferably at least 94%, preferably at least 95%, preferably at least 96%, preferably at least 97%, preferably at least 98%, preferably at least 99%, identity to the respective stretch of the sequence of SEQ ID 1.

Preferably, the term "fragment of progranulin" means a fragment of the full length progranulin comprising a stretch of amino acids not exceeding 592 amino acids of SEQ ID No. 1 or a homolog stretch of amino acids not exceeding 575 amino acids of SEQ ID No. 1 having at least 90%, preferably at least 94%, preferably at least 95%, preferably at least 96%, preferably at least 97%, preferably at least 98%, preferably at least 99%, identity to the sequence of SEQ ID 1.

The term "fragment of progranulin" preferably means a fragment of the mature progranulin comprising a stretch of at least 8 amino acids, preferably at least 18 amino acids, preferably at least 29 amino acids, preferably at least 54 amino acids, at least 68 amino acids, preferably at least 75 amino acids, preferably at least 89 amino acids, preferably at least 105 amino acids, more preferably at least 175 amino acids, of SEQ ID No. 2 or a stretch of at least 8 amino acids, preferably at least 18 amino acids, preferably
at least 29 amino acids, preferably at least 54 amino acids, at least 68 amino acids, preferably at least 75 amino acids, preferably at least 89 amino acids, preferably at least 105 amino acids, more preferably at least 175 amino acids, of SEQ ID No. 2 having at least 90%, preferably at least 94%, preferably at least 95%, preferably at least 96%, preferably at least 97%, preferably at least 98%, preferably at least 99%, identity to the respective stretch of the sequence of SEQ ID 2.

Preferably, the term "fragment of progranulin" means a fragment of the mature progranulin comprising a stretch of amino acids not exceeding 575 amino acids of SEQ ID No. 2 or a homolog stretch of amino acids not exceeding 575 amino acids of SEQ ID No. 2 having at least 90%, preferably at least 94%, preferably at least 95%, preferably at least 96%, preferably at least 97%, preferably at least 98%, preferably at least 99%, identity to the sequence of SEQ ID 2.

Preferably, in the methods of the present invention and/or the use of a test system of the present invention and/or the use of an array of the present invention and/or the use according to the present invention the level of progranulin in an isolated sample of said patient can be detected additionally in combination with the detection a level of at least one protein selected from the group consisting of procalcitonin (PCT), interleukin-2 (IL-2), interleukin-6 (IL-6), interleukin-8 (IL-8), tumor necrosis factor alpha, C-reactive protein (CRP), serum amyloid P component, serum amyloid A, complement factor, mannan-binding lectin, fibrinogen, prothrompin, factor VIII, von Willebrand factor, plasminogen, alpha 2-macroglobulin, ferritin, hepcidin, ceruloplasmin, haptoglobin, alpha-1-acid glycoprotein (AGP), alpha 1-antitrypsin, alpha 1-antichymotrypsin, albumin, retinol-binding protein, antithrombin, transcortin, fragment(s) thereof, homologue(s) thereof and mixtures thereof.

According to the present invention, the term "sample material" is also designated as "sample".

Pursuant to the present invention, the term "biomarker" is meant to designate a protein or protein fragment which is indicative for the incidence of a systemic inflammation, especially a systemic inflammatory response syndrome (SIRS), a sepsis, a severe sepsis or a septic shock.

That means the "biomarker" is used as a means for detecting a systemic inflammation, especially a systemic inflammatory response syndrome (SIRS), a sepsis, a severe sepsis or a septic shock.

The term "individual" or "individuals" as used in the present application covers humans as well as non-human beings such as animals. Preferably, the term "individual" or "individuals" is meant to designate a human being, such as a patient.

The animals are preferably selected from the group consisting of rodents, e.g. mouse, rat, hamster, and other animals, e.g. guinea-pig, rabbit, hare, dog and pig.

These animals can be used to specifically induce certain disease states, like a systemic inflammation, especially a systemic inflammatory response syndrome (SIRS), a sepsis, a severe sepsis or a septic shock, for research purposes. The induction of said disease states can, for example, be effected by treatment of the animals, for example, with radioactive or biological or chemical substances known to induce a systemic inflammation, especially a systemic inflammatory response syndrome (SIRS), a sepsis, a severe sepsis or a septic shock, disease state. The disease states can be induced for example by using bacteria, fungi, viruses, or parasites. The disease states can also be induced using viral transfection systems. It is also possible to use genetically modified animals, in which one or more specific gene function(s) has/have been altered or knock-out animals such as knock-out mice in which a specific gene function has been deleted.

Preferably, the term "healthy individual" or "healthy individuals" is meant to designate an individual(s), not diseased of a systemic inflammation, especially a systemic inflammatory response syndrome (SIRS), a sepsis, a severe sepsis or a septic shock. That is to say, the term "healthy individual(s)" is used only with respect to the pathological condition of a systemic inflammation, especially a systemic inflammatory response syndrome (SIRS), a sepsis, a severe sepsis or a septic shock, and does not exclude the individual(s) to suffer from diseases other than a systemic inflammation, especially a systemic inflammatory response syndrome (SIRS), a sepsis, a severe sepsis or a septic shock.

A healthy individual may have a non-pathological level of progranulin in a sample material.

Preferably, the term "non-pathological level" is meant to designate a maximum level of progranulin that is not harmful to the individual.

Usually, a non-pathological level means that the concentration of progranulin in blood plasma is less than or equal to 250 ng per ml blood plasma, preferably less than or equal to 210 ng per ml blood plasma, preferably less than or equal to 200 ng per ml blood plasma. Preferably a non-pathological level means that the concentration of progranulin in blood plasma is in a range of from 10 to 250 ng per ml blood plasma, preferably in a range of from 30 to 210 ng per ml blood plasma, preferably in a range of from 50 to 200 ng per ml blood plasma.

The reference value for the non-pathological level can be established as a range to be considered as normal, meaning that the person is healthy. The reference value for the non-pathological level can be calculated as the average level of progranulin determined in isolated samples of a plurality of healthy individuals, e.g. of 100 healthy individuals or more.

Preferably, the average non-pathological level means that the concentration of progranulin in blood plasma is less than or equal to 250 ng per ml blood plasma, preferably less than or equal to 210 ng per ml blood plasma, preferably less than or equal to 200 ng per ml blood plasma. Further preferably, the average non-pathological level means that the concentration of progranulin in blood plasma is in a range of from 10 to 250 ng per ml blood plasma, preferably in a range of from 30 to 210 ng per ml blood plasma, preferably in a range of from 50 to 200 ng per ml blood plasma.

In exceptional cases the concentration of progranulin in blood plasma determined in a first sample of an individual not suffering from the pathological condition of a systemic inflammation at a first time might be significantly higher compared to the average non-pathological level. The level of progranulin might be, for example 1,5 to 2,5-fold, higher compared to the average non-pathological level, so that, for example, the level of progranulin in blood plasma might be exceptionally in the range of from 300 to 450 ng per ml blood plasma.

In this case, preferably, at least a second sample isolated from said individual at a second time is provided, wherein said second time is later than said first time, and the level of progranulin in said at least one second sample is determined. An increase of the level of progranulin in said second sample compared to the level of progranulin in said first sample is indicative for presence of a systemic inflammation.

Since the level of progranulin is in these exceptional cases significantly higher compared to the average non-pathological level, the percentage increase of the level of progranulin in said second sample compared to the level of progranulin in said first sample is not as strong in case of a systemic inflammation, compared to a percentage increase based on an average non-pathological level, as mentioned above.

The percentage increase can be 20%, preferably 25%, preferably 40%, preferably 60%, preferably 80%, preferably 100%, preferably 120%, preferably 140%, of the level of progranulin in said second sample compared to the level of progranulin in said first sample in such an exceptional case.

A decrease of the level of progranulin in said second sample compared to the level of progranulin in said first sample is preferably indicative for the course improving.

The term "pathological level" is meant to designate a level of progranulin that is indicative for the pathological condition of a systemic inflammation, especially a systemic inflammatory response syndrome (SIRS), a sepsis, a severe sepsis or a septic shock.

Usually, a pathological level means that the concentration of progranulin in blood plasma is at least 240 ng per ml blood plasma, preferably at least 280 ng per ml blood plasma, preferably at least 300 ng per ml blood plasma, preferably at least 320 ng per ml blood plasma, preferably at least 340 ng per ml blood plasma, preferably at least 360 ng per ml blood plasma, preferably at least 375 ng per ml blood plasma, preferably at least 400 ng per ml blood plasma, preferably at least 440 ng per ll blood plasma, preferably at least 450 ng per ml blood plasma, preferably at least 480 ng per ml blood plasma.

In a preferred embodiment a pathological level means that the concentration of progranulin in blood plasma is 120%, preferably 140%, preferably 160%, preferably 180%, preferably 200%, preferably 220%, preferably 240%, of a non-pathological level, preferably an average non-pathological level.

The reference value for the pathological level can be established as a range to be considered as diseased, meaning that the person suffers from a systemic inflammation, especially a systemic inflammatory response syndrome (SIRS), a sepsis, a severe sepsis or a septic shock. The reference value can be calculated as the average level of progranulin determined in a plurality of isolated samples of individuals suffering from a systemic inflammation, especially a systemic inflammatory response syndrome (SIRS), a sepsis, a severe sepsis or a septic shock.

An increasing level of progranulin may correlate with the severity of a systemic inflammation, especially a systemic inflammatory response syndrome (SIRS), a sepsis, a severe sepsis or a septic shock.

It has been surprisingly discovered by the present inventors that progranulin can be used as biomarker for the detection of a systemic inflammation, especially a systemic inflammatory response syndrome (SIRS), a sepsis, a severe sepsis or a septic shock. The inventors have now surprisingly found that the level of progranulin in a body fluid is elevated in individuals having a systemic inflammation, especially a systemic inflammatory response syndrome (SIRS), a sepsis, a severe sepsis or a septic shock. Furthermore, the level of progranulin in a body fluid can be used to detect a systemic inflammation, especially a systemic inflammatory response syndrome (SIRS), a sepsis, a severe sepsis or a septic shock, in an individual.

Progranulin is a glycoprotein also known as proepithelin, acrogranin, PC-cell-derived growth factor (PCDGF), granulin-epithelin precursor (GEP), 88 kDa glycoprotein (GP88), epithelial transforming growth factor (eTGF) or granulins precursor. It is a 68.5 kDa protein, consisting of 593 amino acids, including a N-terminal signal peptide of 17 amino acids, which appears in vivo in strongly glycosylated form and therefore has a size of approximately 90 kDa, determined by SDS polyacrylamide gel electrophoresis

The mature progranulin, i.e. without signal peptide, is alternatively designated as acrogranin.

Progranulin is expressed in various human tumors, including carcinomas (Cuevas-Antonio R, et al.: Cancer Invest. 2010, pages 452-458; Donald CD et al.: Anticancer Res. 2001, pages 3739-3742; Ong CH and Bateman A.: Histol. Histopathol. 2003, pages 1275-1288), gliomas (Liau LM et al.: Cancer Res. 2000, pages 1353-1360) and sarcomas (Matsumura N et al.: Clin. Cancer Res. 2006, pages 1402-1411)

In the central nervous system (CNS) progranulin is expressed by microglia and neurons, including neocortical and hippocampal pyramid cells as well as Purkinjecells in the cerebellum (Daniel R et al.: J. Histochem. Cytochem. 2000, pages 999-1009; Matsuwaki T et al.: J. Reprod. Dev. 2011, pages 113-119).

Furthermore, it has been shown that a reduction of progranulin leads to frontotemporal lobar degeneration (Cruts M and Van Broeckhoven C: Trends Genet. 2008, pages 186-194).

Serum progranulin concentrations may be associated with macrophage infiltration into omental adipose tissue (Youn BS et al.: Diabetes 2009, pages 627-636).

It has been also shown that proteinase 3 and neutrophil lycocyte elastase enhances inflammation in mice by inactivating anti-inflammatory progranulin (Kessenbrock K et al: J. Clin. Invest. 2008, pages 2438-2447).

Furthermore, a conversion of proepithelin to epithelins has been shown to occur in host defence and wound repair (Zhu J et al.: Cell 2002, pages 867-878).

Progranulin expression has also been shown in advanced human atherosclerotic plaques (Kojima Y et al.: Atherosclerosis 2009, pages 102-108).

The term "progranulin" as used in the present invention means the human progranulin, preferably comprising the amino acid sequences selected from the group consisting of SEQ ID No. 1 and 2.

In another preferred embodiment, said progranulin is a homologue of SEQ ID No. 1 or 2 having at least 90%, preferably at least 94%, preferably at least 95%, preferably at least 96%, preferably at least 97%, preferably at least 98%, preferably at least 99%, identity to the sequence of SEQ ID No. 1 or 2, respectively.

Several biomarkers indicative for systemic inflammation are known in the art, such as C-reactive protein, interleukin-6 or procalcitonin (Riedel S. and Carroll KC. Clin Lab Med. 2013, pages 413-37).

C-reactive protein is an acute-phase protein synthesized in the liver having a biological half life of 19 hours. The level of C-reactive protein is easily determined by detection systems known in the art.

However, in case of hospitalized patients, especially patients requiring intensive care, the level of C-reactive protein is frequently increased. The accuracy for predicting sepsis in these patients is therefore significantly reduced.

Furthermore, the level of C-reactive protein in sample material of patients diagnosed with sepsis increases relatively late in the development of sepsis and persists on high levels for a longer period of time, such as days, which significantly reduces the possibilities for determining the severity of a systemic inflammation and/or the prediction of the survival rate.

Interleukin-6 acts as a pro-inflammatory cytokine and an anti-inflammatory myokine. Interleukin-6 is secreted by T-cells and macrophages to stimulate immune response. The biological half life of interleukin-6 is one hour.

The determination of the level of interleukin-6 allows for a high sensitive and specific diagnosis of a systemic inflammation, especially at early stages.

However, the secretion of interleukin-6 is often down regulated by immune paralysis, a condition in which the immune system is unresponsive. Furthermore, the secretion of interleukin-6 is suppressed by steroids.

Moreover, the plasma level of interleukin-6 reaches its maximum level in a very short period and, together with the relatively low biological half life of one hour, necessitates a precise determination of the level of interleukin-6 in order to diagnose a systemic inflammation.

Procalcitonin is a peptide precursor of the hormone calcitonin. Procalcitonin is composed of 116 amino acids and is produced by T-cells of the troid and by neuroendocrine cells of the lung and the intestines. In serum, procalcitonin has a half life of 20 to 24 hours.

The determination of procalcitonin levels allows for a diagnosis of a systemic inflammation with high specificity.

However, the increase of the level of procalcitonin after the occurrence of a systemic inflammation is delayed. Essays for determining procalcitonin are, furthermore, costly and necessitate a long observation period.

It has been surprisingly discovered by the present inventors that progranulin is a biomarker which allows a faster detection of a systemic inflammation with an increased sensitivity compared to the known biomarkers.

The very early detection of a systemic inflammation, especially a systemic inflammatory response syndrome (SIRS), a sepsis, a severe sepsis or a septic shock, improves significantly the probability of survival of an individual diseased with a systemic inflammation and is a significant improvement for diagnosis of systemic inflammation.

The term "progranulin" as used in the present invention also comprises truncated progranulin, fragments of said progranulin or modified progranulin.

Modifications on protein levels can be due to enzymatic or chemical modifications within the body. For example, the modification can be a glycosylation or phosphorylation or methylation.

Progranulin has seven conserved domains, which are separated by linker sequences. By means of proteolytic cleavage, e.g. catalyzed by serine proteases like e.g. elastase, fragments result, that are called granulines or epithelines.

In a preferred embodiment, said fragment(s) of progranulin comprise at least 90%, preferably at least 94%, preferably at least 95%, preferably at least 96%, preferably at least 97%, preferably at least 98%, preferably at least 99%, sequence identity to SEQ ID No. 1 or 2.

Progranulin is expressed and secreted in particular in strongly proliferating tissues such as adenoid tissue, spleen, skin epithelium, gastrointestinal mucous membranes, haematopoietic cells and in tumor cells.

In a preferred embodiment of the present invention, the level of progranulin is determined by using a detection molecule selected from the group consisting of an antibody against progranulin, an antibody fragment, which specifically binds to an epitope or a suitable structural element of progranulin, a receptor, which specifically binds to an epitope or a suitable structural element of progranulin, and mixtures thereof.

In a preferred embodiment of the present invention, said receptor can be any, preferably proteinacous, structure able to bind specifically to progranulin. The receptor can also be an aptamer. An aptamer can be a DNA or RNA oligonucleotide, having usually 25 to 70 bases, or a peptide aptamer.

In a further preferred embodiment of the present invention, said receptor which specifically binds to an epitope or a suitable structural element of progranulin is a sortilin or a TNF-$\alpha$ receptor.

In a preferred embodiment of the present invention, said antibody against progranulin and/or said antibody fragment, which specifically binds to an epitope or a suitable structural element of progranulin is preferably the monoclonal anti human progranulin antibody PRT1, which is commercially available from Mediagnost Gesellschaft für Forschung und Herstellung von Diagnostika GmbH (Reutlingen, Germany).

In a further preferred embodiment, said antibody against progranulin or said antibody fragment, which specifically binds to an epitope or a suitable structural element of progranulin is a monoclonal, polyclonal or recombinant antibody or antibody fragment.

In a further preferred embodiment, said antibody against progranulin or antibody fragment, which specifically binds to an epitope or a suitable structural element of progranulin, is a modified antibody, such as a humanized antibody, or an antibody fragment, such as an $F(ab')_2$ fragment.

A suitable antibody against progranulin or an antibody fragment, which specifically binds to an epitope or a suitable structural element of progranulin might be raised in a rodent, e.g. mouse, rat, hamster, and other animal, e.g. guinea-pig, cattle, rabbit, hare, dog, pig, goat or sheep, respectively.

In a further preferred embodiment of the present invention said antibody against progranulin or antibody fragment, which specifically binds to an epitope or a suitable structural element of progranulin, detects with high specificity human progranulin, preferably
comprising the amino acid sequences selected from the group consisting of SEQ ID No. 1 and 2.

According to an embodiment of the present invention, the body fluid has been isolated before carrying out the method of the present invention. The methods of the invention are preferably carried out in vitro by a technician in a laboratory or ward testing facility or as point-of-care testing or bedside testing.

Preferably, a rapid assessment of the disease state or the vital status of a patient diagnosed with a systemic inflammation, especially a systemic inflammatory response syndrome (SIRS), a sepsis, a severe sepsis or a septic shock, can be achieved by applying the methods of the present invention at the point of care by personnel without formal laboratory training.

Preferably, the methods of the present invention are carried out together with a blood glucose analysis, an arterial blood gas analysis, an electrolyte analysis, or a combination thereof, in order to asses of the disease state or the vital status of a patient diagnosed with a systemic inflammation, especially a systemic inflammatory response syndrome (SIRS), a sepsis, a severe sepsis or a septic shock.

The methods of the present invention are carried out with sample material such as body fluid which already has been isolated from the human body.

The sample material can be fractionated and/or purified. It is, for example, possible, to store the sample material to be tested in a freezer and to carry out the methods of the present invention at an appropriate point in time after thawing the respective sample material.

In a preferred embodiment, the isolated sample material or sample is a body fluid and selected from the group consisting of blood, blood plasma, serum, lymphatic fluid, cerebro spinal fluid (CSF), amnion fluid, saliva, urine, breast milk and mixtures thereof, preferably blood, blood plasma, serum and mixtures thereof.

According to a preferred embodiment of the invention, a level of progranulin is measured in blood plasma, blood serum or cerebro spinal fluid (CSF). For example, blood serum can be easily obtained by taking blood from an individual to be medically examined and separating the supematant from the clotted blood.

The level of progranulin in said sample material is preferably determined by immunological methods, proteomic techniques, expression analysis, and/or mass spectroscopy, preferably immunological methods, proteomic techniques, and/or mass spectroscopy. The level of progranulin in said sample material is increased.

In a further preferred embodiment of the invention, the level of progranulin in a sample material is significantly elevated, preferably at least 1.5-fold, preferably at least 1.7-fold, preferably at least 2-fold, preferably at least 3-fold, preferably at least 4-fold, further preferably at least 5-fold, increased compared to the level of progranulin in a sample material of an individual not suffering from a systemic inflammation, especially a systemic inflammatory response syndrome (SIRS), a sepsis, a severe sepsis or a septic shock.

In a preferred embodiment of the invention, the level of progranulin in a sample of blood plasma of a healthy individual is less than or equal to 250 ng per ml blood plasma, preferably less than or equal to 210 ng per ml blood plasma, preferably less than or equal to 200 ng per ml blood plasma, which is considered to be a non-pathological level of progranulin.

In a further preferred embodiment of the invention, the level of progranulin in a sample is determined by comparison with at least one sample of a known concentration of progranulin and/or at least one reference
value obtained from measuring at least one sample of a known concentration of progranulin.

Preferably, the at least one reference value is provided as a calibration curve or as a colour chart which are obtained from measuring different concentrations of progranulin for example obtained by serial dilution of a sample of a known concentration of progranulin.

In a preferred embodiment, the level of mRNA encoding progranulin is determined by gene expression analysis using for example nucleic acid probes able to bind to mRNA encoding progranulin being present in a body fluid such as serum. Another preferred embodiment is to produce cDNA by reverse transcription of progranulin mRNA and to specifically detect the amount of respective cDNA. A quantification of the measured mRNA or cDNA, respectively, can be effected by comparison of the measured value with a standard or calibration curve of known amounts of progranulin mRNA or cDNA.

Preferably, said nucleic acid probe can be any natural occurring or synthetic oligonucleotide, as well as cDNA, cRNA and the like, which specifically binds to mRNA encoding progranulin.

The mRNA encoding human progranulin is accessible via:

NCBI accession number: NM_002087.

In another preferred embodiment the present invention allows to monitor the level of progranulin in an isolated sample material or sample over an extended period of time, such as minutes, hours, days or weeks.

Such an extended period of time allows monitoring the individual development and/or progression of a systemic inflammation, especially a systemic inflammatory response syndrome (SIRS), a sepsis, a severe sepsis or a septic shock.

The long-term monitoring, such as, for example, over several days, of the development and/or the course and/or the treatment of a systemic inflammation, especially a systemic inflammatory response syndrome (SIRS), a sepsis, a severe sepsis or a septic shock, over an extended period of time comprises the following steps:

a) providing a first sample isolated from an individual at a first time,
b) determining the level of progranulin in said first sample,
c) providing at least one second sample isolated from the individual at a second time, wherein said second time is later than said first time,
d) determining the level progranulin in said second sample,
e) comparing the determined level of said progranulin in the second sample with the level of said progranulin in the first sample.

In a preferred embodiment, the second sample is isolated from said individual at an appropriate second time, which is later than said first time. The intervals are preferably to be chosen on the basis of the severity of a systemic inflammation, especially a systemic inflammatory response syndrome (SIRS), a sepsis, a severe sepsis or a septic shock. Exemplary points in time may be every hour or every day.

An increase in the level of said progranulin in said second sample compared to the level of said progranulin in said first sample is indicative for the progression of a systemic inflammation, especially a systemic inflammatory response syndrome (SIRS), a sepsis, a severe sepsis or a septic shock, and/or the course worsening and/or the treatment being ineffective.

Due to the long-term monitoring of the development and/or the course and/or the treatment of a systemic inflammation, the present invention, thus, allows for an adaptation of the treatment of a systemic inflammation to the severity of the systemic inflammation and/or the cause of the systemic inflammation.

A decrease in the level of said progranulin in said second sample compared to the level of said progranulin in the first sample is indicative for slowing down of the progression of a systemic inflammation, especially a systemic inflammatory response syndrome (SIRS), a sepsis, a severe sepsis or a septic shock, and/or the course improving and/or the treatment being effective.

The level of said progranulin in said second sample compared to the level of said progranulin in the first sample being essentially constant is also indicative for slowing down of the progression of a systemic inflammation, especially a systemic inflammatory response syndrome (SIRS), a sepsis, a severe sepsis or a septic shock, and/or the course improving and/or the treatment being effective in that there is no worsening.

The term "epitope" is meant to designate any structural element of a protein or peptide or any proteinaceous structure allowing the specific binding of or to an antibody or an antibody fragment, an aptamer, a protein or peptide structure or a receptor.

The present invention provides an early stage biomarker which allows detecting a systemic inflammation, especially a systemic inflammatory response syndrome (SIRS), a sepsis, a severe sepsis or a septic shock, at an early stage. The early detection enables the physician to effectively treat a systemic inflammation, especially a systemic inflammatory response syndrome (SIRS), a sepsis, a severe sepsis or a septic shock, with available medication, which may result in early symptomatic improvement.

An early symptomatic improvement significantly increases the chances of survival of an individual diseased with a systemic inflammation, especially a systemic inflammatory response syndrome (SIRS), a sepsis, a severe sepsis or a septic shock.

Moreover, the present invention allows monitoring the level of progranulin in a body fluid such as blood serum over an extended period of time, such as weeks.

The long term monitoring of the level of progranulin allows evaluating the progression of a systemic inflammation, especially a systemic inflammatory response syndrome (SIRS), a sepsis, a severe sepsis or a septic shock. The level of progranulin can be routinely checked, for example, every day. If an increase of the level of progranulin is detected, this can be indicative for a progression of a systemic inflammation, especially a systemic inflammatory response syndrome (SIRS), a sepsis, a severe sepsis or a septic shock, and/or the course worsening and/or the treatment being ineffective.

The long term monitoring of the level of progranulin also allows for the detection of a recurrence of a systemic inflammation, especially a systemic inflammatory response syndrome (SIRS), a sepsis, a severe sepsis or a septic shock, after a treatment.

Moreover, the course of the disease and/or the treatment can be monitored. If the level of progranulin further increases, for example after medication, this can be indicative for exacerbation of the pathological condition.

That means the level of progranutin is a valuable clinical parameter for detecting and/or monitoring of a systemic inflammation, especially a systemic inflammatory response syndrome (SIRS), a sepsis, a severe sepsis or a septic shock.

The level of progranulin in body fluids might be elevated without the presence of symptoms required for the clinical diagnosis of a systemic inflammation, especially a systemic inflammatory response syndrome (SIRS), a sepsis, a severe sepsis or a septic shock. Therefore, the level of progranulin is an important clinical parameter to allow an early diagnosis and, consequently, an early treatment of a systemic inflammation, especially a systemic inflammatory response syndrome (SIRS), a sepsis, a severe sepsis or a septic shock.

The method of the invention for detection of a systemic inflammation, especially a systemic inflammatory response syndrome (SIRS), a sepsis, a severe sepsis or a septic shock, comprises the step of providing an isolated sample material which has been taken from an individual, then determining the level of progranulin in the isolated sample material, and finally comparing the determined level of said progranulin with one or more reference values. In one embodiment, one or more further biomarker(s) is/are additionally detected in an isolated sample material which has been taken from an individual, the level of the biomarker(s) is/are determined and compared with one or more respective reference values.

The reference value can be calculated as the average level of progranulin determined in a plurality of isolated samples of healthy individuals or individuals suffering from a systemic inflammation, especially a systemic inflammatory response syndrome (SIRS), a sepsis, a severe sepsis or a septic shock. This or these reference value(s) can be established as a range to be considered as normal meaning that the person is healthy or suffers from a systemic inflammation, especially a systemic inflammatory response syndrome (SIRS), a sepsis, a severe sepsis or a septic shock. A threshold value within a range can then be indicative for healthy condition or the pathological condition of a systemic inflammation, especially a systemic inflammatory response syndrome (SIRS), a sepsis, a severe sepsis or a septic shock. A range of reference value can be established by taking a statistically
relevant number of body fluid samples, such as serum samples, of healthy individuals as it is done for any other medical parameter range such as, e.g., blood sugar. Preferably, two reference values are calculated which are designated as negative control and positive control 1. The reference value of the negative control is calculated from healthy individuals and the positive control is calculated from individuals suffering from a systemic inflammation, especially a systemic inflammatory response syndrome (SIRS), a sepsis, a severe sepsis or a septic shock.

In an another embodiment of the present invention, the reference value(s) can be individual reference value(s) calculated as the average level of progranulin determined in a plurality of isolated samples taken from the individual over a period of time.

When monitoring the level of progranulin over an extended period of time, such as hours, days or weeks, it is possible to establish an individual average level. The level of progranulin can be measured, for example, from the same blood serum sample when measuring blood sugar and can be used to specifically detect any individual increase of the level of progranulin.

The present invention is particularly useful in terms of personalized medicine.

The reference value(s) for further biomarkers can also be calculated in the same way as described for the level of progranulin. The average level(s) of progranulin further biomarkers may be the mean or median level.

Another aspect of the present invention is the use of a test system for detecting a systemic inflammation, especially a systemic inflammatory response syndrome (SIRS), a sepsis, a severe sepsis or a septic shock, in an isolated sample material of an individual comprising:

a) at least one detection molecule, which specifically recognizes progranulin,
b) a reagent for detecting the binding of progranulin to said at least one detection molecule, and
c)optionally, a solid support which supports said at least one detection molecule.

In a preferred embodiment, said at least one detection molecule is bound to said solid support such as, e.g., a particularly plastic, surface or beads to allow binding and detection of progranulin. For example, a conventional microtiter plate can be used as a plastic surface.

The detection of the binding of at least one detection molecule can be effected, for example, by using a secondary antibody labeled with a detectable group. The detectable group can be, for example, a radioactive isotope or an enzyme like horseradish peroxidase or alkaline phosphatase detectable by adding a suitable substrate to produce, for example, a colour or a fluorescence signal.

The test system to be used can be an immunoassay such as an enzyme-linked immunosorbentassay (ELISA) or a radio immunoassay (RIA) or luminescence immunossay (LIA). However, any other immunological test system using the specificity of antibodies or fragments of antibodies or receptors can be used such as Western blotting or immuno precipitation or flow cytometry.

In a preferred embodiment, the test system to be used can be a gene expression analysis assay comprising for example nucleic acid probes able to bind to mRNA encoding progranulin being present in a body fluid such as serum. Another preferred embodiment is to produce cDNA by reverse transcription of progranulin mRNA and to specifically detect the amount of respective cDNA with said test system. A quantification of the measured mRNA or cDNA, respectively, can be effected by comparison of the measured value with a standard or calibration curve of known amounts of progranulin mRNA or cDNA.

Preferably, said nucleic acid probe can be any natural occurring or synthetic oligonucleotide, as well as cDNA, cRNA and the like, which specifically binds to mRNA encoding progranulin.

The test system to be used can be designed as an array comprising detection molecules for detecting a systemic inflammation, especially a systemic inflammatory response syndrome (SIRS), a sepsis, a severe sepsis or a septic shock, in an individual, wherein the detection molecule can be:

- an antibody or antibody fragment immobilized on a solid support for binding to and detecting of an epitope of progranulin, or
- a receptor immobilized on a solid support for binding to and detecting of progranulin, or
- a nucleic acid probe immobilized on a solid support for binding to mRNA encoding progranulin.

In a preferred embodiment of the test system to be used said array comprises at least one antibody against progranulin and/or at least one antibody fragment, which specifically recognizes progranulin, which is immobilized on a solid support and specifically recognizes and binds progranulin, in said isolated sample.

The detection molecule can be an antibody against progranulin and/or at least one antibody fragment, which specifically recognizes progranulin to which a detectable moiety, such as a dye, preferable fluorescence, enzyme, etc., is coupled.

It is apparent that there are several possibilities using primary and, if necessary or appropriate, secondary antibodies and or antibody fragments to produce an immunological test system.

Preferably, the array comprises further detection molecules which are biomarkers for detecting a systemic inflammation, especially a systemic inflammatory response syndrome (SIRS), a sepsis, a severe sepsis or a septic shock.

The term "immobilized" as used in the present invention is meant to designate direct or indirect, via at least one linker or spacer molecule, attachment to a solid support.

For example, indirect immobilization can be affected by using a synthetic oligonucleotide such as an aptamer. Aptamers are single-stranded oligonucleotides that assume a specific, sequence-dependent shape and bind to protein targets with high specificity and affinity. Aptamers are identified using the SELEX process (Tuerk C. and Gold L. (1990) Science 249: 505-510; Ellington AD and Szostak JW. (1990) Nature 346: 818-822).

Alternatively, the present invention also comprises an inverse array comprising patient samples immobilized on a solid support which can be detected by the above defined detection molecules.

Preferably the array comprises detection molecules which are immobilized to a solid surface at identifiable positions.

The term "array" as used in the present invention refers to a grouping or an arrangement of the test system, without being necessarily a regular arrangement. An array comprises preferably at least 2 or more sets of the above defined detection molecules or samples of individuals to be tested. Preferably, the array comprises at least 50 or more sets of detection molecules or samples of individuals to be tested, further preferred at least 100 or more sets of detection molecules or samples of individuals to be tested. Preferably, the array comprises at least 500 or more sets of the above defined detection molecules or samples of individuals to be tested.

Preferably, the array can be either a micro array or a macro array.

In a preferred embodiment, the above defined detection molecules are immobilised to a solid surface or support or solid support surface. This array or microarray is then screened by contacting the array with proteinaceous probes prepared from samples of individuals to be tested.

In another preferred embodiment, the proteinaceous probes prepared from samples of individuals to be tested are immobilised to a solid surface or support or solid support surface. This array or microarray is then screened by contacting the array with the above defined detection molecules.

The support can be a polymeric material such as nylon or plastic or an inorganic material such as silicon, for example a silicon wafer, or ceramic. Pursuant to a preferred embodiment, glass ($SiO_2$) is used as solid support material. The glass can be a glass slide or glass chip. Pursuant to another embodiment of the invention the glass substrate has an atomically flat surface.

For example, the array can be comprised of:

- an antibody and/or antibody fragment immobilized on a solid support for binding to and detecting of an epitope of progranulin, or
- a receptor immobilized on a solid support for binding to and detecting of an epitope of progranulin, or
- a nucleic acid probe immobilized on a solid support for binding to and detecting of mRNA encoding progranulin.

Preferably, different amounts of detection molecules are immobilized each on the solid support to allow an accurate quantification of the level of progranulin.

Pursuant to another embodiment of the invention, the level of progranulin is determined by mass spectroscopy.

Mass spectroscopy allows to specifically detect the level of progranulin very easily.

Any suitable ionization method in the field of mass spectroscopy known in the art can be employed to ionize progranulin. The ionization methods comprise electron impact (EI), chemical ionization (CI), field ionization (FDI), electrospray ionization (ESI), laser desorption ionization (LDI), matrix assisted laser desorption ionization (MALDI) and surface enhanced laser desorption ionization (SELDI).

Any suitable detection method in the field of mass spectroscopy known in the art can be employed to determined the molecular mass of progranulin. The detection methods comprise quadrupol mass spectroscopy (QMS), fourier transform mass spectroscopy (FT-MS) and time-of-flight mass spectroscopy (TOF-MS).

In an embodiment of the present invention the sensitivity and/or specificity of the detection of a systemic inflammation, especially a systemic inflammatory response syndrome (SIRS), a sepsis, a severe sepsis or a septic shock, is enhanced by additionally detection of a level of a further biomarker. In particular, in one embodiment the sensitivity and/or specificity of the detection of a systemic inflammation, especially a systemic inflammatory response syndrome (SIRS), a sepsis, a severe sepsis or a septic shock, is enhanced by detection of a level of another protein in combination with the level of progranulin.

Preferably, the sensitivity and specifity of the methods, arrays, test systems and uses according to the present invention are increased by the combination of detecting the level of progranulin.

The sensitivity and specificity are defined as follow:

The sensitivity is the number of true positive patients (%) with regard to the number of all patients (100 %). The patients are individuals having a systemic inflammation, especially a systemic inflammatory response syndrome (SIRS), a sepsis, a severe sepsis or a septic shock.

The specificity is the number of true negative individuals (%) with regard to the number of all healthy individuals (100%).

The sensitivity and specificity can be alternatively defined by the following formulas:

| | | diagnosis | |
|---|---|---|---|
| | | + | - |
| test | + | TP | FP |
| | - | FN | TN |

TP: True positive (test positive, diagnosis correct);
FP: False positive (test positive, diagnosis incorrect);
TN: True negative (test negative, diagnosis correct);
FN: False negative (test negative, diagnosis incorrect);

The sensitivity is calculated by the following formula:

$$TP/(TP + FN)$$

and the specificity is calculated by the following formula:

$$TN/ (TN + FP)$$

The result of each analysis group, which is selected from TP, FP, TN, FN, is calculated for a plurality of isolated samples selected from the group consisting of healthy individuals, patients suffering from a systemic inflammation, especially a systemic inflammatory response syndrome (SIRS), a sepsis, a severe sepsis or a septic shock. TP, FP, TN, FN relates to number of individuals that are correlated with the status true positive, false positive, true negative, false negative, respectively.

The methods of the present invention can be carried out in combination with other diagnostic methods for detection of a systemic inflammation, especially a systemic inflammatory response syndrome (SIRS), a sepsis, a severe sepsis or a septic shock, to increase the overall sensitivity and/or specificity.

The detection of the level of progranulin allows a very early detection of a systemic inflammation, especially a systemic inflammatory response syndrome (SIRS), a sepsis, a severe sepsis or a septic shock, and can therefore be used as a very early marker.

Preferably, the methods of the present invention are carried out as an early detection and/or monitoring method. If the results of the methods of the present invention should indicate the incidence of a systemic inflammation, especially a systemic inflammatory response syndrome (SIRS), a sepsis, a severe sepsis or a septic shock, further examinations, in particular desired evaluations, should be carried out.

The present invention further provides a method for determining whether a compound is effective in the treatment of a systemic inflammation, especially a systemic inflammatory response syndrome (SIRS), a sepsis, a severe sepsis or a septic shock.

The method for determining whether a compound is effective in the treatment of a systemic inflammation, especially a systemic inflammatory response syndrome (SIRS), a sepsis, a severe sepsis or a septic shock, comprises the steps of:

a) providing an isolated sample from an individual diseased of systemic inflammation and treated with a compound,
b) determining the level of progranulin in said isolated sample of said individual, and
c) comparing the determined level of progranulin with one or more reference values.

In another embodiment, the method for determining whether a compound is effective in the treatment of a systemic inflammation, especially a systemic inflammatory response syndrome (SIRS), a sepsis, a severe sepsis or a septic shock, comprises the steps of:

a) treating of an individual diseased of a systemic inflammation, especially a systemic inflammatory response syndrome (SIRS), a sepsis, a severe sepsis or a septic shock, with said compound,
b) determining the level of progranulin in said sample of said patient, and
c) comparing the determined level of said progranulin with one or more reference values.

A "compound" is any pharmaceutically active agent for treating a systemic inflammation, especially a systemic inflammatory response syndrome (SIRS), a sepsis, a severe sepsis or a septic shock. For example the "compound" may be preferably be one or more chemical substances, an antibody, protein, peptide, antisense mRNA, small molecular drug, or combinations thereof.

The level of progranulin in a sample material of said patient can be determined by the above described detection techniques.

## Literature


Cuevas-Antonio R, Cancino C, Arechavaleta-Velasco F, et as. Expression of progranulin (Acrogranin/PCDGF/Granulin-Epithelin Precursor) in benign and malignant ovarian tumors and activation of MAPK signaling in ovarian cancer cell line. Cancer Invest. Jun 2010;28(5):452-458.

Donald CD, Laddu A, Chandham P, et al. Expression of progranulin and the epithelin/granulin precursor acrogranin correlates with neoplastic state in renal epithelium. Anticancer Res. Nov-Dec 2001; 21(6A):3739-3742.

Ong CH, Bateman A. Progranulin (granulin-epitheiin precursor, PC-cell derived growth factor, acrogranin) in proliferation and tumorigenesis. Histol Histopathol. Oct 2003; 18(4):1275-1288.

Liau LM, Lallone RL, Seitz RS, et al. Identification of a human glioma-associated growth factor gene, granulin, using differential immuno-absorption. Cancer Res. Mar 1 2000; 60(5):1353-1360.

Matsumura N, Mandai M, Miyanishi M, et al. Oncogenic property of acrogranin in human uterine leiomyosarcoma: direct evidence of genetic contribution in in vivo tumorigenesis. Clin Cancer Res. Mar 1 2006;12(5):1402-1419.

Daniel R, He Z, Carmichael KP, Halper J, Bateman A. Cellular localization of gene expression for progranulin. J Histochem Cytochem. Jul 2000;48(7):999-1009.

Matsuwaki T, Asakura R, Suzuki M, Yamanouchi K, Nishihara M. Age-dependent changes in progranulin expression in the mouse brain. J Reprod Dev. Feb 2011;57(1):113-119.

Cruts M, Van Broeckhoven C. Loss of progranulin function in frontotemporal lobar degeneration. Trends Genet. Apr 2008;24(4):186-194.

Youn BS, Bang SI, Kloting N, et al. Serum progranulin concentrations may be associated with macrophage infiltration into omental adipose tissue. Diabetes. Mar 2009;58(3):627-636.

Kessenbrock K, Frohlich L, Sixt M, et al. Proteinase 3 and neutrophil elastase enhance inflammation in mice by inactivating antiinflammatory progranulin. J. Clin: Invest. Jul 2008;118(7):2438-2447.

Zhu J, Nathan C, Jin W, et al. Conversion of proepithelin to epithelins: roles of SLPI and elastase in host defense and wound repair. Cell. Dec 13 2002; 111 (6):867-878.
Kojima Y, Ono K, Inoue K, et al. Progranulin expression in advanced human atherosclerotic plaque. Atherosclerosis. Sep 2009; 206(1):102-108.
Riedel S., Carroll KC. Laboratory detection of sepsis: biomarkers and molecular approaches. Clin Lab Med. Sep 2013;33(3):413-37.
American College of Chest Physicians/Society of Critical Care Medicine Consensus Conference: definitions for sepsis and organ failure and guidelines for the use of innovative therapies in sepsis. Crit Care Med. Jun 1992;20(6):864-874.
Bone RC, Sprung CL, Sibbald WJ. Definitions for sepsis and organ failure. Crit Care Med. Jun 1892;20(6):724-726.

## Sequences

SEQ ID No. 1 corresponds to the full length progranulin of the indicated species, including a signal peptide.
SEQ ID No. 2 corresponds to the mature progranulin of the indicated species without a signal peptide.
SEQ ID No. 3 corresponds to the CRNA encoding progranulin of the indicated species.
The following Figures and Examples are given for illustrative purpose only. The invention is not to be construed to be limited to the following examples:

## Figures:

Figure 1 depicts serum progranulin levels in all included individuals diagnosed with a sepsis (n=10), individuals diagnosed with a severe sepsis (n=10) and individuals diagnosed with a septic shock (n=20) and in control individuals, with mean and standard error of the mean indicated.

Figure 2 shows a receiver operating characteristic (ROC) curve obtained by plotting the sensitivity versus 1 - the specificity using a serum progranilin cutoff level of 170 % of the progranulin plasma level of healthy control individuals to classify individuals diseased of a systemic inflammation versus control individuals.

Figure 3 shows the amino acid sequence of human progranulin deposited as NCBI Reference Sequence: NP_002078.1. The amino acid sequence of progranulin consists of 593 amino acids including a N-terminal signal peptide which is denoted by underlining.

Figure 4 shows the mRNA sequence encoding human progranulin deposited as NCBI Reference Sequence: NM_002087.2.

## Example 1:

Unless other way stated, the Example was carried out following the protocol of the manufacturer of the analytical systems.
The respective progranulin levels in human plasma were determined using a commercially available enzyme-linked immunosorbent assay for quantitative determination of human progranulin, human progranulin ELISA 103, obtained from Mediagnost Gesellschaft für Forschung und Herstellung von Diagnostika GmbH (Reutlingen, Germany),.
The respective human plasma samples were obtained from healthy control individuals (n=10), patients diagnosed with a sepsis (n=10), patients diagnosed with a severe sepsis (n=10) and patients diagnosed with a septic shock (n=20).
The respective patients were diagnosed according to the criteria defined by the ACCP/SCCM consensus conference definitions for sepsis or organ failure (American College of Chest Physicians/Society of Critical Care Medicine Consensus Conference: Crit Care Med. 1992, pages 864-874; Bone RC et al.: Crit Care Med. 1992, pages 724-726).
Samples were analysed in triplicate.
The absorbance was measured at a wavelength of 450 nm (reference filter set to 590 nm) with a commercially available ELISA reader (Mulfiskan™ FC Microplate Photometer, Thermo Fisher Scientific, Waltham, USA).
The progranulin plasma levels determined are depicted in Fig. 1. The respective progranulin plasma levels are depicted as percentage increase compared to the progranulin plasma level of healthy individuals, which is set to 100% and which corresponds to a progranulin plasma level of 200 ng per ml.
A Kruskal-Wallis one-way analysis of variance by ranks was used for comparing of the respective subgroups of patients.

Fig. 2 shows a receiver operating characteristic (ROC) curve obtained by plotting the fraction of true positives out of the total actual positives versus the fraction of the false positive out of the total actual negatives. A serum progranilin cutoff level of 170 % of the progranulin plasma level of healthy control individuals was used to classify individuals diseased of a systemic inflammation versus control individuals.

An area-under-the-curve (AUC) statistic was computed.

## Claims

1. A method for detecting a systemic inflammation comprising:

   a) providing an isolated sample which has been taken from an individual,
   b) determining a pathological level of progranulin in said isolated sample.

2. A method of monitoring the development and/or the course and/or the treatment of a systemic inflammation comprising:

   a) providing a first sample isolated from an individual at a first time,
   b) determining a level of progranulin in said first sample,
   c) providing at least a second sample isolated from said individual at a second time, wherein said second time is later than said first time,
   d) determining the level of progranulin in said isolated sample,
   e) comparing the determined level of progranulin in said second sample and the level of progranulin in said first sample,

   wherein an increase of the level of progranulin in said second sample compared to the level of progranulin in said first sample is indicative for the progression of a systemic inflammation, and/or the course worsening and/or the treatment being ineffective, and
   wherein a decrease of the level of progranulin in said second sample compared to the level of progranulin in said first sample is indicative for slowing down of the progression of a systemic inflammation, and/or the course improving and/or the treatment being effective.

**3.** The method of claim 1, wherein said level of progranulin in said sample is determined by immunological methods, proteomics techniques, gene expression analysis, and/or mass spectroscopy.

**4.** The method of claim 2, wherein said level of progranulin in said first sample and/or said second sample is determined by immunological methods, proteomics techniques, gene expression analysis, and/or mass spectroscopy.

**5.** The method of any of claims 1 to 4, wherein said systemic inflammation is a systemic inflammatory response syndrome (SIRS), a sepsis, a severe sepsis or a septic shock.

**6.** The method of any of claims 1 to 5, wherein said sample is selected from the group consisting of blood, blood plasma, serum, lymphatic fluid, cerebro spinal fluid (CSF), amnion fluid, saliva, urine, breast milk and mixtures thereof.

**7.** The method of any of claims 1 to 6, wherein the level of progranulin in said sample, preferably blood plasma, is 120% of a non-pathological level calculated as the average level of progranulin determined in isolated samples of a plurality of individuals not diseased of a systemic inflammation.

**8.** The method of any of claims 1 to 7, wherein said progranulin comprises the amino acid sequences selected from the group consisting of SEQ ID No. 1 and 2 .

**9.** The method of claim 8, wherein said progranulin is a homolog of SEQ ID No. 1 or 2 having at least 90% identity to the sequence of SEQ ID No. 1 or 2, respectively.

**10.** The method of any of claims 1 to 9, wherein additionally a level of at least one protein selected from the group consisting of procalcitonin (PCT), interleukin-2 (IL-2), interleukin-6 (IL-6), interleukin-8 (IL-8), tumor necrosis factor alpha, C-reactive protein (CRP), serum amyloid P component, serum amyloid A, complement factor, mannan-binding lectin, fibrinogen, prothrompin, factor VIII, von Willebrand factor, plasminogen, alpha 2-macroglobulin, ferritin, hepcidin, ceruloplasmin, haptoglobin, alpha-1-acid glycoprotein (AGP), alpha 1-antitrypsin, alpha 1-antichymotrypsin, albumin, retinol-binding protein, antithrombin, transcortin and mixtures thereof is determined.

**11.** Use of a test system for detecting a systemic inflammation in an isolated sample of an individual comprising:

a) at least one detection molecule, which specifically recognizes progranulin,
b) a reagent for detecting the binding of progranulin to said at least one detection molecule, and
c) optionally, a solid support which supports said at least one detection molecule.

**12.** Use of a test system according to claim 11, wherein said test system is a gene expression analysis assay or an immunoassay, preferably an enzyme-linked immunosorbentassay (ELISA), a radio immunoassay (RIA), a luminescence immunossay (LIA), a Western blott, an immuno precipitation, a flow cytometry or a biochip.

**13.** A method for determining whether a compound is effective in the treatment of a systemic inflammation comprising:

a) providing an isolated sample from an individual diseased of systemic inflammation and treated with a compound,
b) determining the level of progranulin in said isolated sample of said individual, and
c) comparing the determined level of progranulin with one or more reference values.

**14.** Progranulin for use as a biomarker for a systemic inflammation.

**15.** Progranulin according to claim 14, wherein said systemic inflammation is a systemic inflammatory response syndrome (SIRS), a sepsis, a severe sepsis or a septic shock.

## Patentansprüche

**1.** Verfahren zum Nachweisen einer systemischen Entzündung, umfassend:

a) das Bereitstellen einer isolierten Probe, die einem Individuum entnommen worden ist, und
b) das Bestimmen einer pathologischen Konzentration von Progranulin in der isolierten Probe.

2. Verfahren zum Überwachen der Entwicklung und/oder des Verlaufs und/oder der Behandlung einer systemischen Entzündung, umfassend:

a) das Bereitstellen einer ersten Probe, die von einem Individuum zu einem ersten Zeitpunkt isoliert worden ist,
b) das Bestimmen der Konzentration von Progranulin in der ersten Probe,
c) das Bereitstellen mindestens einer zweiten Probe, die vom Individuum zu einem zweiten Zeitpunkt isoliert worden ist, wobei der zweite Zeitpunkt später als der erste Zeitpunkt ist,
d) das Bestimmen der Konzentration von Progranulin in der isolierten Probe und
e) das Vergleichen der in der zweiten Probe bestimmten Konzentration von Progranulin mit der Konzentration von Progranulin in der ersten Probe,

wobei eine Zunahme der Konzentration von Progranulin in der zweiten Probe im Vergleich zur Konzentration von Progranulin in der ersten Probe ein Indiz dafür ist, dass die systemische Entzündung fortschreitet und/oder dass sich der Verlauf verschlechtert und/oder dass die Behandlung unwirksam ist, und
wobei eine Abnahme der Konzentration von Progranulin in der zweiten Probe im Vergleich zur Konzentration von Progranulin in der ersten Probe ein Indiz dafür ist, dass sich das Fortschreiten der systemischen Entzündung verlangsamt und/oder dass sich der Verlauf verbessert und/oder dass die Behandlung wirksam ist.

3. Verfahren nach Anspruch 1, wobei die Konzentration von Progranulin in der Probe durch immunologische Verfahren, proteomische Techniken, Genexpressionsanalyse und/oder Massenspektroskopie bestimmt wird.

4. Verfahren nach Anspruch 2, wobei die Konzentration von Progranulin in der ersten Probe und/oder der zweiten Probe durch immunologische Verfahren, proteomische Techniken, Genexpressionsanalyse und/oder Massenspektroskopie bestimmt wird.

5. Verfahren nach einem der Ansprüche 1 bis 4, wobei es sich bei der systemischen Entzündung um ein systemisches inflammatorisches Response-Syndrom (SIRS), eine Sepsis, eine schwere Sepsis oder einen septischen Schock handelt.

6. Verfahren nach einem der Ansprüche 1 bis 5, wobei die Probe aus der Gruppe ausgewählt wird, die aus Blut, Blutplasma, Serum, lymphatischer Flüssigkeit, zerebrospinaler Flüssigkeit (CSF), Amnionflüssigkeit, Speichel, Urin, Muttermilch und Gemischen davon besteht.

7. Verfahren nach einem der Ansprüche 1 bis 6, wobei die Konzentration von Progranulin in der Probe, vorzugsweise Blutplasma, 120 % einer nicht-pathologischen Konzentration beträgt die berechnet wird als durchschnittliche Konzentration von Progranulin in Proben, die von einer Mehrzahl von Individuen, die nicht an einer systmischen Entzündung leiden, isoliert worden sind.

8. Verfahren nach einem der Ansprüche 1 bis 7, wobei das Progranulin die Aminosäuresequenzen umfasst, die aus der Gruppe ausgewählt sind, die aus SEQ ID NO: 1 und 2 besteht.

9. Verfahren nach Anspruch 8, wobei es sich beim Progranulin um ein Homologes von SEQ ID NO: 1 oder 2 handelt, das seine mindestens 90 %-ige Identität mit der Sequenz von SEQ ID NO: 1 bzw. 2 aufweist.

10. Verfahren nach einem der Ansprüche 1 bis 9, wobei zusätzlich die Konzentration mindestens eines Proteins bestimmt wird, das aus der Gruppe ausgewählt ist, die besteht aus Procalcitonin (PCT), Interleukin-2 (IL-2), Interleukin-6 (IL-6), Interleukin-8 (IL-8), Tumornekrosefaktor-alpha, C-reaktivem Protein (CRP), Serum-Amyolid P-Komponente, SerumAmyloid A, Komplementfaktor, Mannan-bindendes Lectin, Fibrinogen, Prothrombin, Faktor VIII, von Willebrand-Faktor, Plasminogen, alpha-2-Makroglobulin, Ferritin, Hepcidin, Ceruloplasmin, Haptoglobin, alpha-1-Säure-Glycoprotein (AGP), alpha-1-Antitrypsin, alpha-1-Antichymotrypsin, Albumin, Retinol-bindendes Protein, Antithrombin, Transcortin und Gemischen davon.

11. Verwendung eines Testsystems zum Nachweisen einer systemischen Entzündung in einer isolierten Probe eines Individuums, das Folgendes umfasst:

a) mindestens ein Nachweismolekül, das spezifisch Progranulin erkennt,
b) ein Reagenz zum Nachweisen der Bindung von Progranulin an das mindestens eine Nachweismolekül und
c) gegebenenfalls einen festen Träger, der das mindestens eine Nachweismolekül trägt.

**12.** Verwendung eines Testsystems nach Anspruch 11, wobei es sich beim Testsystem um einen Genexpressionsanalyse-Test oder einen Immunoassay, insbesondere einen enzymgekoppelten Immunosorbensassay (ELISA), einen Radioimmunoassay (RIA), einen Lumineszenz-Immunoassay (LIA), einen Western-Blot, eine Immunopräzipitation, eine Durchflusszytometrie oder einen Biochip handelt.

**13.** Verfahren zum Bestimmen, ob eine Verbindung bei der Behandlung einer systemischen Entzündung wirksam ist, umfassend:

a) das Bereitstellen einer isolierten Probe von einem Individuum, das an einer systemischen Entzündung erkrankt ist und mit einer Verbindung behandelt worden ist,
b) das Bestimmen der Konzentration von Progranulin in der isolierten Probe des Individuums und
c) das Vergleichen der bestimmten Konzentration von Progranulin mit einem oder mehreren Referenzwerten.

**14.** Progranulin zur Verwendung als Biomarker für eine systemische Entzündung.

**15.** Progranulin nach Anspruch 14, wobei es sich bei der systemischen Entzündung um ein systemisches inflammatorisches Response-Syndrom (SIRS), eine Sepsis, eine schwere Sepsis oder einen septischen Schock handelt.

## Revendications

**1.** Procédé pour détecter une inflammation systémique comprenant :

a) la mise à disposition d'un échantillon isolé qui a été prélevé chez un individu,
b) la détermination d'un taux pathologique de progranuline dans ledit échantillon isolé.

**2.** Procédé de surveillance du développement et/ou de l'évolution et/ou du traitement d'une inflammation systémique comprenant :

a) la mise à disposition d'un premier échantillon isolé à partir d'un individu à un premier moment,
b) la détermination d'un taux de progranuline dans ledit premier échantillon,
c) la mise à disposition d'au moins un deuxième échantillon isolé à partir dudit individu à un deuxième moment, où ledit deuxième moment est ultérieur audit premier moment,
d) la détermination du taux de progranuline dans ledit échantillon isolé,
e) la comparaison du taux de progranuline déterminé dans ledit deuxième échantillon et du taux de progranuline dans ledit premier échantillon,

où une augmentation du taux de progranuline dans ledit deuxième échantillon par comparaison aux taux de progranuline dans ledit premier échantillon est indicative de la progression d'une inflammation systémique, et/ou de l'aggravation de l'évolution et/ou de l'inefficacité du traitement, et
où une réduction du taux de progranuline dans ledit deuxième échantillon par comparaison aux taux de progranuline dans ledit premier échantillon est indicative du ralentissement de la progression d'une inflammation systémique, et/ou de l'amélioration de l'évolution et/ou de l'efficacité du traitement.

**3.** Procédé selon la revendication 1, dans lequel ledit taux de progranuline dans ledit échantillon est déterminé par des procédés immunologiques, des techniques de protéomique, une analyse d'expression génique, et/ou la spectroscopie de masse.

**4.** Procédé selon la revendication 2, dans lequel ledit taux de progranuline dans ledit premier échantillon et/ou ledit deuxième échantillon est déterminé par des procédés immunologiques, des techniques de protéomique, une analyse d'expression génique, et/ou la spectroscopie de masse.

**5.** Procédé selon l'une quelconque des revendications 1 à 4, dans lequel ladite inflammation systémique est un syndrome de réponse inflammatoire systémique (SIRS), un sepsis, un sepsis sévère ou un choc septique.

**6.** Procédé selon l'une quelconque des revendications 1 à 5, dans lequel ledit échantillon est sélectionné dans le groupe consistant en du sang, du plasma sanguin, du sérum, du liquide lymphatique, du liquide céphalo-rachidien (LCR), du liquide amniotique, de la salive, de l'urine, du lait maternel et des mélanges de ceux-ci.

**7.** Procédé selon l'une quelconque des revendications 1 à 6, dans lequel le taux de progranuline dans ledit échantillon, de préférence du plasma sanguin, est 120 % d'un taux non pathologique calculé en tant que taux moyen de progranuline déterminé dans des isolats cliniques d'une pluralité d'individus ne souffrant pas d'inflammation systémique.

**8.** Procédé selon l'une quelconque des revendications 1 à 7, dans lequel ladite progranuline comprend les séquences d'acides aminés sélectionnées dans le groupe consistant en SEQ ID NO: 1 et 2.

**9.** Procédé selon la revendication 8, dans lequel ladite progranuline est un homologue de SEQ ID NO: 1 ou 2 présentant au moins 90 % d'identité avec la séquence de SEQ ID NO: 1 ou 2, respectivement.

**10.** Procédé selon l'une quelconque des revendications 1 à 9, dans lequel en outre un taux d'au moins une protéine sélectionnée dans le groupe consistant en la procalcitonine (PCT), l'interleukine-2 (IL-2), l'interleukine-6 (IL-6), l'interleukine-8 (IL-8), le facteur de nécrose tumorale alpha, la protéine C réactive (CRP), le composant sérique amyloïde P, l'amyloïde sérique A, le facteur du complément, la lectine liant le mannane, le fibrinogène, la prothrombine, le facteur VIII, le facteur von Willebrand, le plasminogène, l'alpha-2 macroglobuline, la ferritine, l'hepcidine, la céruloplasmine, l'haptoglobine, l'alpha-1-glycoprotéine acide (AGP), l'alpha-1 antitrypsine, l'alpha-1 antichymotrypsine, l'albumine, la protéine liant le rétinol, l'antithrombine, la transcortine et des mélanges de celles-ci, est déterminé.

**11.** Utilisation d'un système de test pour détecter une inflammation systémique dans un échantillon isolé d'un individu comprenant :

a) au moins une molécule de détection, qui reconnaît spécifiquement la progranuline,
b) un réactif pour détecter la liaison de la progranuline à ladite au moins une molécule de détection, et
c) facultativement, un support solide qui porte ladite au moins une molécule de détection.

**12.** Utilisation d'un système de test selon la revendication 11, dans laquelle ledit système de test est un essai d'analyse d'expression génique ou un dosage immunologique, de préférence un dosage d'immunoabsorption par enzyme liée (ELISA), un dosage radioimmunologique (RIA), un dosage immunologique par luminescence (LIA), un transfert de Western, une immunoprécipitation, la cytométrie en flux ou une biopuce.

**13.** Procédé pour déterminer si un composé est efficace dans le traitement d'une inflammation systémique comprenant :

a) la mise à disposition d'un échantillon isolé à partir d'un individu souffrant d'une inflammation systémique et traité avec un composé,
b) la détermination du taux de progranuline dans ledit échantillon isolé dudit individu, et
c) la comparaison du taux de progranuline déterminé avec une ou plusieurs valeurs de référence.

**14.** Progranuline destinée à être utilisée en tant que biomarqueur d'une inflammation systémique.

**15.** Progranuline selon la revendication 14, où ladite inflammation systémique est un syndrome de réponse inflammatoire systémique (SIRS), un sepsis, un sepsis sévère ou un choc septique.

Figure 1:

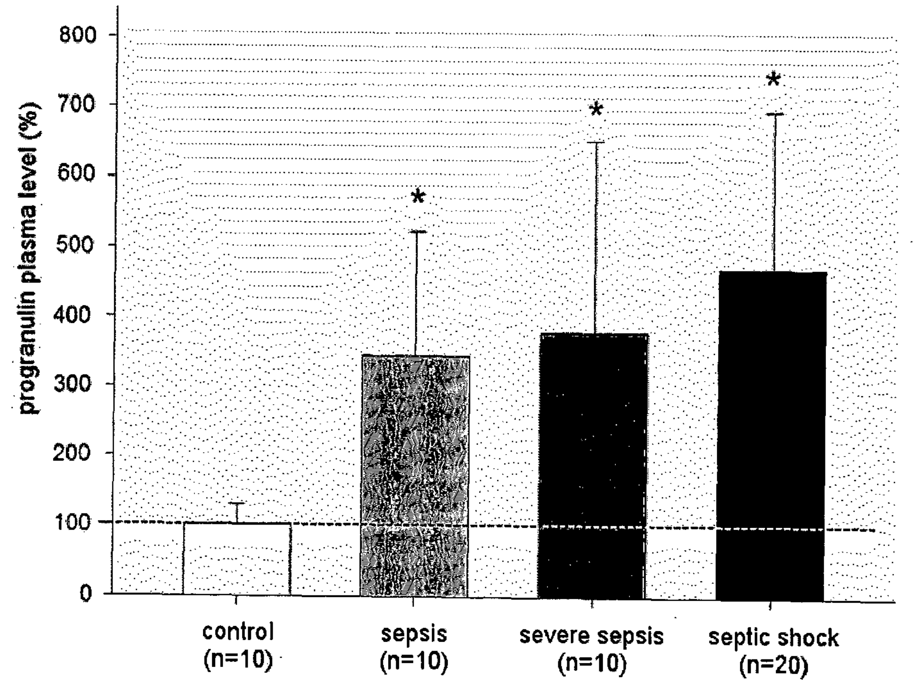


* p<0.01, Kruskal-Wallis One Way Analysis of Variance on Ranks, All Pairwise Multiple Comparison Procedures (Dunn's Method)

Figure 2:

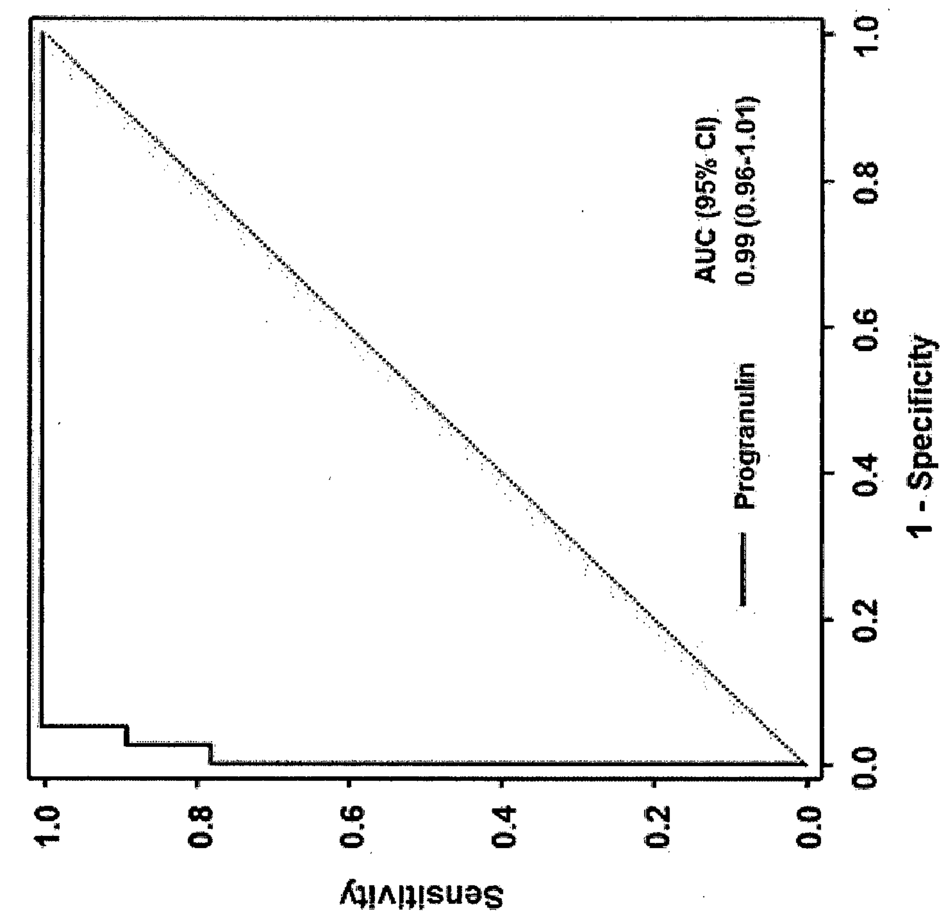
Sensitivity
1.0
0.8
0.6
0.4
0.2
0.0
1 - Specificity
0.0
0.2
0.4
0.6
0.8
1.0
Progranulin
AUC (95% CI)
0.99 (0.96-1.01)

Figure 3:

NCBI Reference Sequence: NP_002078.1

Progranulin (granulins precursor)

Organism: Homo sapiens

```
  1  MWTLVSWVAL TAGLVAGTRC PDGQFCPVAC CLDPGGASYS CCRPLLDKWP TTLSRHLGGP
 61  CQVDAHCSAG HSCIFTVSGT SSCCPFPEAV ACGDGHHCCP RGFHCSADGR SCFQRSGNNS
121  VGAIQCPDSQ FECPDFSTCC VMVDGSWGCC PMPQASCCED RVHCCPHGAF CDLVHTRCIT
181  PTGTHPLAKK LPAQRTNRAV ALSSSVMCPD ARSRCPDGST CCELPSGKYG CCPMPNATCC
241  SDHLHCCPQD TVCDLIQSKC LSKENATTDL LTKLPAHTVG DVKCDMEVSC PDGYTCCRLQ
301  SGAWGCCPFT QAVCCEDHIH CCPAGFTCDT QKGTCEQGPH QVPWMEKAPA HLSLPDPQAL
361  KRDVPCDNVS SCPSSDTCCQ LTSGEWGCCP IPEAVCCSDH QHCCPQGYTC VAEGQCQRGS
421  EIVAGLEKMP ARRASLSHPR DIGCDQHTSC PVGQTCCPSL GGSWACCQLP HAVCCEDRQH
481  CCPAGYTCNV KARSCEKEVV SAQPATFLAR SPHVGVKDVE CGEGHFCHDN QTCCRDNRQG
541  WACCPYRQGV CCADRRHCCP AGFRCAARGT KCLRREAPRW DAPLRDPALR QLL
```

Figure 4:

NCBI Reference Sequence: NM_002087.2

Progranulin (granulins precursor) mRNA

Organism: Homo sapiens

```
   1 ggcgagagga agcagggagg agagtgattt gagtagaaaa gaaacacagc attccaggct
  61 ggccccacct ctatattgat aagtagccaa tgggagcggg tagccctgat ccctggccaa
 121 tggaaactga ggtaggcggg tcatcgcgct ggggtctgta gtctgagcgc tacccggttg
 181 ctgctgccca aggaccgcgg agtcggacgc aggcagacca tgtggaccct ggtgagctgg
 241 gtggccttaa cagcagggct ggtggctgga acgcggtgcc cagatggtca gttctgccct
 301 gtggcctgct gcctggaccc cggaggagcc agctacagct gctgccgtcc ccttctggac
 361 aaatggccca caacactgag caggcatctg ggtggcccct gccaggttga tgcccactgc
 421 tctgccggcc actcctgcat ctttaccgtc tcagggactt ccagttgctg ccccttccca
 481 gaggccgtgg catgcgggga tggccatcac tgctgcccac ggggcttcca ctgcagtgca
 541 gacgggcgat cctgcttcca aagatcaggt aacaactccg tgggtgccat ccagtgccct
 601 gatagtcagt tcgaatgccc ggacttctcc acgtgctgtg ttatggtcga tggctcctgg
 661 gggtgctgcc ccatgcccca ggcttcctgc tgtgaagaca gggtgcactg ctgtccgcac
 721 ggtgccttct gcgacctggt tcacacccgc tgcatcacac ccacgggcac ccacccccctg
 781 gcaaagaagc tccctgccca gaggactaac agggcagtgg ccttgtccag ctcggtcatg
 841 tgtccggacg cacggtcccg gtgccctgat ggttctacct gctgtgagct gcccagtggg
 901 aagtatggct gctgcccaat gcccaacgcc acctgctgct ccgatcacct gcactgctgc
 961 ccccaagaca ctgtgtgtga cctgatccag agtaagtgcc tctccaagga gaacgctacc
1021 acggacctcc tcactaagct gcctgcgcac acagtggggg atgtgaaatg tgacatggag
1081 gtgagctgcc cagatggcta tacctgctgc cgtctacagt cgggggcctg gggctgctgc
1141 ccttttaccc aggctgtgtg ctgtgaggac cacatacact gctgtcccgc ggggtttacg
1201 tgtgacacgc agaagggtac ctgtgaacag gggccccacc aggtgccctg gatggagaag
1261 gccccagctc acctcagcct gccagaccca caagccttga agagagatgt cccctgtgat
1321 aatgtcagca gctgtccctc ctccgatacc tgctgccaac tcacgtctgg ggagtggggc
1381 tgctgtccaa tcccagaggc tgtctgctgc tcggaccacc agcactgctg cccccagggc
1441 tacacgtgtg tagctgaggg gcagtgtcag cgaggaagcg agatcgtggc tggactggag
1501 aagatgcctg cccgccgggc ttccttatcc caccccagag acatcggctg tgaccagcac
1561 accagctgcc cggtggggca gacctgctgc ccgagcctgg gtgggagctg ggcctgctgc
1621 cagttgcccc atgctgtgtg ctgcgaggat cgccagcact gctgcccggc tggctacacc
1681 tgcaacgtga aggctcgatc ctgcgagaag gaagtggtct ctgcccagcc tgccaccttc
1741 ctggcccgta gccctcacgt gggtgtgaag gacgtggagt gtggggaagg acacttctgc
1801 catgataacc agacctgctg ccgagacaac cgacagggct gggcctgctg tccctaccgc
1861 cagggcgtct gttgtgctga tcggcgccac tgctgtcctg ctggcttccg ctgcgcagcc
1921 aggggtacca agtgtttgcg cagggaggcc ccgcgctggg acgccccttt gagggaccca
1981 gccttgagac agctgctgtg agggacagta ctgaagactc tgcagccctc gggaccccac
2041 tcggagggtg ccctctgctc aggcctccct agcacctccc cctaaccaaa ttctccctgg
2101 accccattct gagctcccca tcaccatggg aggtggggcc tcaatctaag gccttccctg
2161 tcagaagggg gttgtggcaa aagccacatt acaagctgcc atcccctccc cgtttcagtg
2221 gaccctgtgg ccaggtgctt ttccctatcc acagggtgt ttgtgtgtgt gcgcgtgtgc
2281 gtttcaataa agtttgtaca ctttcaaaaa aaaaaaaaaa aaa
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Crit Care Med. American College of Chest Physicians/Society of Critical Care Medicine Consensus Conference, 1992, 864-874
- **BONE RC et al.** *Crit Care Med.,* 1992, 724-726
- **CUEVAS-ANTONIO R et al.** *Cancer Invest.,* 2010, 452-458
- **DONALD CD et al.** *Anticancer Res.,* 2001, 3739-3742
- **ONG CH ; BATEMAN A.** *Histol. Histopathol.,* 2003, 1275-1288
- **LIAU LM et al.** *Cancer Res.,* 2000, 1353-1360
- **MATSUMURA N et al.** *Clin. Cancer Res.,* 2006, 1402-1411
- **DANIEL R et al.** *J. Histochem. Cytochem.,* 2000, 999-1009
- **MATSUWAKI T et al.** *J. Reprod. Dev.,* 2011, 113-119
- **CRUTS M ; VAN BROECKHOVEN C.** *Trends Genet.,* 2008, 186-194
- **YOUN BS et al.** *Diabetes,* 2009, 627-636
- **KESSENBROCK K et al.** *J. Clin. Invest.,* 2008, 2438-2447
- **ZHU J et al.** *Cell,* 2002, 867-878
- **KOJIMA Y et al.** *Atherosclerosis,* 2009, 102-108
- **RIEDEL S. ; CARROLL KC.** *Clin Lab Med.,* 2013, 413-37
- **TUERK C. ; GOLD L.** *Science,* 1990, vol. 249, 505-510
- **ELLINGTON AD ; SZOSTAK JW.** *Nature,* 1990, vol. 346, 818-822
- **CUEVAS-ANTONIO R ; CANCINO C ; ARECHAVALETA-VELASCO F.** Expression of progranulin (Acrogranin/PCDGF/Granulin-Epithelin Precursor) in benign and malignant ovarian tumors and activation of MAPK signaling in ovarian cancer cell line. *Cancer Invest.,* June 2010, vol. 28 (5), 452-458
- **DONALD CD ; LADDU A ; CHANDHAM P et al.** Expression of progranulin and the epithelin/granulin precursor acrogranin correlates with neoplastic state in renal epithelium. *Anticancer Res.,* November 2001, vol. 21 (6A), 3739-3742
- **ONG CH ; BATEMAN A.** Progranulin (granulin-epitheiin precursor, PC-cell derived growth factor, acrogranin) in proliferation and tumorigenesis. *Histol Histopathol.,* October 2003, vol. 18 (4), 1275-1288
- **LIAU LM ; LALLONE RL ; SEITZ RS et al.** Identification of a human glioma-associated growth factor gene, granulin, using differential immuno-absorption. *Cancer Res.,* 01 March 2000, vol. 60 (5), 1353-1360
- **MATSUMURA N ; MANDAI M ; MIYANISHI M et al.** Oncogenic property of acrogranin in human uterine leiomyosarcoma: direct evidence of genetic contribution in in vivo tumorigenesis. *Clin Cancer Res.,* 01 March 2006, vol. 12 (5), 1402-1419
- **DANIEL R ; HE Z ; CARMICHAEL KP ; HALPER J ; BATEMAN A.** Cellular localization of gene expression for progranulin. *J Histochem Cytochem.,* July 2000, vol. 48 (7), 999-1009
- **MATSUWAKI T ; ASAKURA R ; SUZUKI M ; YAMANOUCHI K ; NISHIHARA M.** Age-dependent changes in progranulin expression in the mouse brain. *J Reprod Dev.,* February 2011, vol. 57 (1), 113-119
- **CRUTS M ; VAN BROECKHOVEN C.** Loss of progranulin function in frontotemporal lobar degeneration. *Trends Genet.,* April 2008, vol. 24 (4), 186-194
- **YOUN BS ; BANG SI ; KLOTING N et al.** Serum progranulin concentrations may be associated with macrophage infiltration into omental adipose tissue. *Diabetes,* March 2009, vol. 58 (3), 627-636
- **KESSENBROCK K ; FROHLICH L ; SIXT M et al.** Proteinase 3 and neutrophil elastase enhance inflammation in mice by inactivating antiinflammatory progranulin. *J. Clin: Invest.,* July 2008, vol. 118 (7), 2438-2447
- **ZHU J ; NATHAN C ; JIN W et al.** Conversion of proepithelin to epithelins: roles of SLPI and elastase in host defense and wound repair. *Cell,* 13 December 2002, vol. 111 (6), 867-878
- **KOJIMA Y ; ONO K ; INOUE K et al.** Progranulin expression in advanced human atherosclerotic plaque. *Atherosclerosis,* September 2009, vol. 206 (1), 102-108
- **RIEDEL S. ; CARROLL KC.** Laboratory detection of sepsis: biomarkers and molecular approaches. *Clin Lab Med.,* September 2013, vol. 33 (3), 413-37
- American College of Chest Physicians/Society of Critical Care Medicine Consensus Conference: definitions for sepsis and organ failure and guidelines for the use of innovative therapies in sepsis. *Crit Care Med.,* June 1992, vol. 20 (6), 864-874

- **BONE RC ; SPRUNG CL ; SIBBALD WJ.** Definitions for sepsis and organ failure. *Crit Care Med.,* June 1892, vol. 20 (6), 724-726
- *American College of Chest Physicians/Society of Critical Care Medicine Consensus Conference: Crit Care Med.,* 1992, 864-874

SEQUENCE LISTING

<110> Mediagnost Gesellschaft für Forschung und Herstellung von Diagnostika GmbH

<120> Method for detecting a systemic inflamation and test system

<130> 52236

<160> 3

<170> PatentIn version 3.5

<210> 1
<211> 593
<212> PRT
<213> Homo sapiens

<220>
<221> SIGNAL
<222> (1).. (17)
<223> Signalpeptide

<400> 1

```
Met Trp Thr Leu Val Ser Trp Val Ala Leu Thr Ala Gly Leu Val Ala
1               5                   10                  15

Gly Thr Arg Cys Pro Asp Gly Gln Phe Cys Pro Val Ala Cys Cys Leu
            20                  25                  30

Asp Pro Gly Gly Ala Ser Tyr Ser Cys Cys Arg Pro Leu Leu Asp Lys
        35                  40                  45

Trp Pro Thr Thr Leu Ser Arg His Leu Gly Gly Pro Cys Gln Val Asp
    50                  55                  60

Ala His Cys Ser Ala Gly His Ser Cys Ile Phe Thr Val Ser Gly Thr
65                  70                  75                  80

Ser Ser Cys Cys Pro Phe Pro Glu Ala Val Ala Cys Gly Asp Gly His
                85                  90                  95

His Cys Cys Pro Arg Gly Phe His Cys Ser Ala Asp Gly Arg Ser Cys
            100                 105                 110

Phe Gln Arg Ser Gly Asn Asn Ser Val Gly Ala Ile Gln Cys Pro Asp
        115                 120                 125

Ser Gln Phe Glu Cys Pro Asp Phe Ser Thr Cys Cys Val Met Val Asp
    130                 135                 140

Gly Ser Trp Gly Cys Cys Pro Met Pro Gln Ala Ser Cys Cys Glu Asp
```

```
145                 150                 155                 160

Arg Val His Cys Cys Pro His Gly Ala Phe Cys Asp Leu Val His Thr
                165                 170                 175

Arg Cys Ile Thr Pro Thr Gly Thr His Pro Leu Ala Lys Lys Leu Pro
            180                 185                 190

Ala Gln Arg Thr Asn Arg Ala Val Ala Leu Ser Ser Ser Val Met Cys
        195                 200                 205

Pro Asp Ala Arg Ser Arg Cys Pro Asp Gly Ser Thr Cys Cys Glu Leu
    210                 215                 220

Pro Ser Gly Lys Tyr Gly Cys Cys Pro Met Pro Asn Ala Thr Cys Cys
225                 230                 235                 240

Ser Asp His Leu His Cys Cys Pro Gln Asp Thr Val Cys Asp Leu Ile
                245                 250                 255

Gln Ser Lys Cys Leu Ser Lys Glu Asn Ala Thr Thr Asp Leu Leu Thr
            260                 265                 270

Lys Leu Pro Ala His Thr Val Gly Asp Val Lys Cys Asp Met Glu Val
        275                 280                 285

Ser Cys Pro Asp Gly Tyr Thr Cys Cys Arg Leu Gln Ser Gly Ala Trp
    290                 295                 300

Gly Cys Cys Pro Phe Thr Gln Ala Val Cys Cys Glu Asp His Ile His
305                 310                 315                 320

Cys Cys Pro Ala Gly Phe Thr Cys Asp Thr Gln Lys Gly Thr Cys Glu
                325                 330                 335

Gln Gly Pro His Gln Val Pro Trp Met Glu Lys Ala Pro Ala His Leu
            340                 345                 350

Ser Leu Pro Asp Pro Gln Ala Leu Lys Arg Asp Val Pro Cys Asp Asn
        355                 360                 365

Val Ser Ser Cys Pro Ser Ser Asp Thr Cys Cys Gln Leu Thr Ser Gly
    370                 375                 380

Glu Trp Gly Cys Cys Pro Ile Pro Glu Ala Val Cys Cys Ser Asp His
385                 390                 395                 400
```

```
Gln His Cys Cys Pro Gln Gly Tyr Thr Cys Val Ala Glu Gly Gln Cys
                405                 410                 415

Gln Arg Gly Ser Glu Ile Val Ala Gly Leu Glu Lys Met Pro Ala Arg
            420                 425                 430

Arg Ala Ser Leu Ser His Pro Arg Asp Ile Gly Cys Asp Gln His Thr
        435                 440                 445

Ser Cys Pro Val Gly Gln Thr Cys Cys Pro Ser Leu Gly Gly Ser Trp
    450                 455                 460

Ala Cys Cys Gln Leu Pro His Ala Val Cys Cys Glu Asp Arg Gln His
465                 470                 475                 480

Cys Cys Pro Ala Gly Tyr Thr Cys Asn Val Lys Ala Arg Ser Cys Glu
                485                 490                 495

Lys Glu Val Val Ser Ala Gln Pro Ala Thr Phe Leu Ala Arg Ser Pro
            500                 505                 510

His Val Gly Val Lys Asp Val Glu Cys Gly Glu Gly His Phe Cys His
        515                 520                 525

Asp Asn Gln Thr Cys Cys Arg Asp Asn Arg Gln Gly Trp Ala Cys Cys
    530                 535                 540

Pro Tyr Arg Gln Gly Val Cys Cys Ala Asp Arg Arg His Cys Cys Pro
545                 550                 555                 560

Ala Gly Phe Arg Cys Ala Ala Arg Gly Thr Lys Cys Leu Arg Arg Glu
                565                 570                 575

Ala Pro Arg Trp Asp Ala Pro Leu Arg Asp Pro Ala Leu Arg Gln Leu
            580                 585                 590

Leu
```

<210> 2
<211> 576
<212> PRT
<213> Homo sapiens

<400> 2

```
Thr Arg Cys Pro Asp Gly Gln Phe Cys Pro Val Ala Cys Cys Leu Asp
1               5                   10                  15
```

```
Pro Gly Gly Ala Ser Tyr Ser Cys Cys Arg Pro Leu Leu Asp Lys Trp
            20                  25                  30

Pro Thr Thr Leu Ser Arg His Leu Gly Gly Pro Cys Gln Val Asp Ala
        35                  40                  45

His Cys Ser Ala Gly His Ser Cys Ile Phe Thr Val Ser Gly Thr Ser
    50                  55                  60

Ser Cys Cys Pro Phe Pro Glu Ala Val Ala Cys Gly Asp Gly His His
65                  70                  75                  80

Cys Cys Pro Arg Gly Phe His Cys Ser Ala Asp Gly Arg Ser Cys Phe
                85                  90                  95

Gln Arg Ser Gly Asn Asn Ser Val Gly Ala Ile Gln Cys Pro Asp Ser
            100                 105                 110

Gln Phe Glu Cys Pro Asp Phe Ser Thr Cys Cys Val Met Val Asp Gly
        115                 120                 125

Ser Trp Gly Cys Cys Pro Met Pro Gln Ala Ser Cys Cys Glu Asp Arg
    130                 135                 140

Val His Cys Cys Pro His Gly Ala Phe Cys Asp Leu Val His Thr Arg
145                 150                 155                 160

Cys Ile Thr Pro Thr Gly Thr His Pro Leu Ala Lys Lys Leu Pro Ala
                165                 170                 175

Gln Arg Thr Asn Arg Ala Val Ala Leu Ser Ser Ser Val Met Cys Pro
            180                 185                 190

Asp Ala Arg Ser Arg Cys Pro Asp Gly Ser Thr Cys Cys Glu Leu Pro
        195                 200                 205

Ser Gly Lys Tyr Gly Cys Cys Pro Met Pro Asn Ala Thr Cys Cys Ser
    210                 215                 220

Asp His Leu His Cys Cys Pro Gln Asp Thr Val Cys Asp Leu Ile Gln
225                 230                 235                 240

Ser Lys Cys Leu Ser Lys Glu Asn Ala Thr Thr Asp Leu Leu Thr Lys
                245                 250                 255

Leu Pro Ala His Thr Val Gly Asp Val Lys Cys Asp Met Glu Val Ser
            260                 265                 270
```

Cys Pro Asp Gly Tyr Thr Cys Cys Arg Leu Gln Ser Gly Ala Trp Gly
275 280 285

Cys Cys Pro Phe Thr Gln Ala Val Cys Cys Glu Asp His Ile His Cys
290 295 300

Cys Pro Ala Gly Phe Thr Cys Asp Thr Gln Lys Gly Thr Cys Glu Gln
305 310 315 320

Gly Pro His Gln Val Pro Trp Met Glu Lys Ala Pro Ala His Leu Ser
325 330 335

Leu Pro Asp Pro Gln Ala Leu Lys Arg Asp Val Pro Cys Asp Asn Val
340 345 350

Ser Ser Cys Pro Ser Ser Asp Thr Cys Cys Gln Leu Thr Ser Gly Glu
355 360 365

Trp Gly Cys Cys Pro Ile Pro Glu Ala Val Cys Cys Ser Asp His Gln
370 375 380

His Cys Cys Pro Gln Gly Tyr Thr Cys Val Ala Glu Gly Gln Cys Gln
385 390 395 400

Arg Gly Ser Glu Ile Val Ala Gly Leu Glu Lys Met Pro Ala Arg Arg
405 410 415

Ala Ser Leu Ser His Pro Arg Asp Ile Gly Cys Asp Gln His Thr Ser
420 425 430

Cys Pro Val Gly Gln Thr Cys Cys Pro Ser Leu Gly Gly Ser Trp Ala
435 440 445

Cys Cys Gln Leu Pro His Ala Val Cys Cys Glu Asp Arg Gln His Cys
450 455 460

Cys Pro Ala Gly Tyr Thr Cys Asn Val Lys Ala Arg Ser Cys Glu Lys
465 470 475 480

Glu Val Val Ser Ala Gln Pro Ala Thr Phe Leu Ala Arg Ser Pro His
485 490 495

Val Gly Val Lys Asp Val Glu Cys Gly Glu Gly His Phe Cys His Asp
500 505 510

Asn Gln Thr Cys Cys Arg Asp Asn Arg Gln Gly Trp Ala Cys Cys Pro
515 520 525

Tyr Arg Gln Gly Val Cys Cys Ala Asp Arg Arg His Cys Cys Pro Ala
530 535 540

Gly Phe Arg Cys Ala Ala Arg Gly Thr Lys Cys Leu Arg Arg Glu Ala
545 550 555 560

Pro Arg Trp Asp Ala Pro Leu Arg Asp Pro Ala Leu Arg Gln Leu Leu
565 570 575

<210> 3
<211> 2323
<212> DNA
<213> Homo sapiens

<400> 3

ggcgagagga agcagggagg agagtgattt gagtagaaaa gaaacacagc attccaggct 60
ggccccacct ctatattgat aagtagccaa tgggagcggg tagccctgat ccctggccaa 120
tggaaactga ggtaggcggg tcatcgcgct ggggtctgta gtctgagcgc tacccggttg 180
ctgctgccca aggaccgcgg agtcggacgc aggcagacca tgtggaccct ggtgagctgg 240
gtggccttaa cagcagggct ggtggctgga acgcggtgcc cagatggtca gttctgccct 300
gtggcctgct gcctggaccc cggaggagcc agctacagct gctgccgtcc ccttctggac 360
aaatggccca caacactgag caggcatctg ggtggcccct gccaggttga tgcccactgc 420
tctgccggcc actcctgcat ctttaccgtc tcagggactt ccagttgctg cccctcccca 480
gaggccgtgg catgcgggga tggccatcac tgctgcccac ggggcttcca ctgcagtgca 540
gacgggcgat cctgcttcca aagatcaggt aacaactccg tgggtgccat ccagtgccct 600
gatagtcagt tcgaatgccc ggacttctcc acgtgctgtg ttatggtcga tggctcctgg 660
gggtgctgcc ccatgcccca ggcttcctgc tgtgaagaca gggtgcactg ctgtccgcac 720
ggtgccttct gcgacctggt tcacacccgc tgcatcacac ccacgggcac ccacccccctg 780
gcaaagaagc tccctgccca gaggactaac agggcagtgg ccttgtccag ctcggtcatg 840
tgtccggacg cacggtcccg gtgccctgat ggttctacct gctgtgagct gcccagtggg 900
aagtatggct gctgcccaat gcccaacgcc acctgctgct ccgatcacct gcactgctgc 960
ccccaagaca ctgtgtgtga cctgatccag agtaagtgcc tctccaagga gaacgctacc 1020
acggacctcc tcactaagct gcctgcgcac acagtggggg atgtgaaatg tgacatggag 1080
gtgagctgcc cagatggcta tacctgctgc cgtctacagt cgggggcctg ggctgctgc 1140
ccttttaccc aggctgtgtg ctgtgaggac cacatacact gctgtcccgc ggggtttacg 1200
tgtgacacgc agaagggtac ctgtgaacag ggccccacc aggtgccctg gatggagaag 1260
gccccagctc acctcagcct gccagaccca caagccttga agagagatgt cccctgtgat 1320

```
aatgtcagca gctgtccctc ctccgatacc tgctgccaac tcacgtctgg ggagtggggc   1380
tgctgtccaa tcccagaggc tgtctgctgc tcggaccacc agcactgctg cccccagggc   1440
tacacgtgtg tagctgaggg gcagtgtcag cgaggaagcg agatcgtggc tggactggag   1500
aagatgcctg cccgccgggc ttccttatcc caccccagag acatcggctg tgaccagcac   1560
accagctgcc cggtggggca gacctgctgc ccgagcctgg gtgggagctg ggcctgctgc   1620
cagttgcccc atgctgtgtg ctgcgaggat cgccagcact gctgcccggc tggctacacc   1680
tgcaacgtga aggctcgatc ctgcgagaag gaagtggtct ctgcccagcc tgccaccttc   1740
ctggcccgta gccctcacgt gggtgtgaag gacgtggagt gtggggaagg acacttctgc   1800
catgataacc agacctgctg ccgagacaac cgacagggct gggcctgctg tccctaccgc   1860
cagggcgtct gttgtgctga tcggcgccac tgctgtcctg ctggcttccg ctgcgcagcc   1920
aggggtacca agtgtttgcg cagggaggcc ccgcgctggg acgccccttt gagggaccca   1980
gccttgagac agctgctgtg agggacagta ctgaagactc tgcagccctc gggaccccac   2040
tcggagggtg ccctctgctc aggcctccct agcacctccc cctaaccaaa ttctccctgg   2100
accccattct gagctcccca tcaccatggg aggtggggcc tcaatctaag gccttccctg   2160
tcagaagggg gttgtggcaa aagccacatt acaagctgcc atcccctccc cgtttcagtg   2220
gaccctgtgg ccaggtgctt ttccctatcc acaggggtgt ttgtgtgtgt gcgcgtgtgc   2280
gtttcaataa agtttgtaca ctttcaaaaa aaaaaaaaaa aaa                     2323
```